# EUROPEAN PATENT APPLICATION

(11) **EP 2 311 976 A1**
(43) Date of publication of application: **20.04.2011**
(21) Application number: 09173437.6
(22) Date of filing: 19.10.2009
(51) Int. Cl.: C12Q 1/68

(54) **Method for Streptococcus Pneumoniae diagnosis and serotyping**

(71) Applicant: Azienda Ospedaliero-Universitaria Meyer, 50139 Firenze (IT)
(72) Inventor: Azzari, Chiara, 50139 Firenze (IT); Moriondo, Maria, 50139 Firenze (IT); Resti, Massimo, 50139 Firenze (IT)
(74) Representative: Capasso, Olga

(57) **Abstract**

Method for detecting a specific serogroup of *S. pneumoniae* in a biological sample obtained from a subject, comprising the following steps:
a) extracting *S. pneumoniae* DNA directly from the sample;
b) incubating the extracted DNA under conditions such as to enable the amplification of at least one serogroup specific target region comprised in SEQ ID No. 64 of the *cps locus* of *S. pneumoniae*, if present in the sample and
c) detecting the amplified product.

## Description

### FIELD OF THE INVENTION

The present invention relates to a method for the typing of multiple Pneumococcal serotypes directly on clinical samples. The method is based on realtime PCR amplification of specific genomic regions by using identified combinations of primer/probe sets.

### BACKGROUND ART

At present, what is known about pneumococcal serotype distribution in invasive infections or carriage, is based mostly on cultural isolate typing [24].

Pneumococcal serogroup and serotype identification is currently performed by using large panels of expensive antisera by capsular swelling reaction [9,10,13]. However, cross-reactions between serotypes can occur and some strains are difficult to serotype or remain non-serotypable [11-14]. At present, capsule thickness is a serious problem in serologic serotyping. Antisera cannot react correctly with capsular structures of certain pneumococcal serotypes such as serotype 3. This problem can be overcome using the method of the present invention since DNA extraction and amplification is not disturbed by capsule thickness. Serotyping of isolates by molecular methods such as multiplex sequential PCR (MS-PCR) has been previously performed [2] and high sensitivity and excellent concordance with standard cultural methods has been demonstrated [2]. However, it is well known that cultures may give false-negative results because of small sample volume, previous antibiotic therapy, or unsatisfactory conditions of transport and storage which can impair the viability of pathogens. In all the cases where culture growth is not obtained, serotyping cannot be performed neither with Quellung reaction nor with molecular methods. The authors of the present invention previously demonstrated that MS-PCR can be used directly on clinical samples for *Streptococcus Pneumoniae* serotyping, thus giving results where cultural methods fail [3]. The method disclosed in the application WO 2009/024844 used multiplex sequential PCR directly on clinical sample for the diagnosis and serotyping of Streptococcus pneumoniae. The method has a sensitivity and specificity significantly higher than standard cultural methods known, at that time, as the gold standard for microbiological diagnosis. The present invention aims at further improving the diagnosis and serotyping of Streptococcus pneumoniae infections.

MS-PCR techniques can be further improved using realtime PCR. At present, among molecular methods, realtime PCR has probably the best performance in terms of sensitivity, specificity and rapidity. The use of specific procedures and primer/probes sets makes the test extremely specific and sensitive. Moreover, detection of amplification products during the amplification step itself makes the test the most rapid in molecular biology. As for sensitivity, it has been previously demonstrated in different research fields that realtime PCR sensitivity is higher than that of standard PCR and evaluation on agarose gel [19]. In fact, realtime PCR has been used over a large spectrum of fields in biological research [25-27]. In recent years, molecular techniques have been applied successfully to the identification of infectious agents. The knowledge of DNA/RNA sequences of most bacteria allow to obtain an etiologic diagnosis and bacterial serotyping [1, 2] even where standard culture methods fail [3, 4]. The need for viable bacteria as well as technical expertise requirements are serious drawbacks of the culture detection-system. In addiction, autolysis in culture media and antibiotic therapy often performed on an outpatient basis at the beginning of febrile illnesses, make diagnosis and serotyping with culture methods more difficult [4, 5].

Molecular methods, based on DNA detection, do not require viable bacteria so that bacterial detection can be obtained even after starting antimicrobic treatment [4]. For this reason molecular methods have higher sensitivity in reaching an etiologic diagnosis, greatly reduce the proportion of false-negative results and can be used as a better tools to obtain an epidemiologic picture in different areas in the world.

Among the most common bacterial pathogens, *Streptococcus Pneumoniae* is considered the leading cause of invasive bacterial infections both in children and in elderly people [6]. The organism causes at least 1.6 million deaths each year and is responsible for several invasive infections [7].

Capsular polysaccharide helps to differentiate 91 distinct serotypes of *Streptococcus Pneumoniae* [8]. Pneumococcal serotyping is currently performed by using capsular swelling (Quellung) reaction, considered the traditional 'gold standard', [9-10]. However, cross-reactions between serotypes can occur and some strains remain non-serotypable [11-14]. At present, strains with a thick capsule such as serotype 3 cannot be easily typed because specific antisera are prevented from reaching specific epitopes on the capsule itself. This problem can be overcome using molecular methods since DNA extraction and amplification is not disturbed by capsule thickness. Effective vaccines induce specific anti-capsular antibodies [15]. The available vaccine used in pediatric age induces protection against 7 major serotypes and new vaccines against 10 or 13 serotypes will be available in the next future. Individuating serotypes causing invasive infections is extremely important in order to understand pneumococcal epidemiology, plan correct programs of vaccination and evaluate the impact of the existing program. In particular, the correct analysis of serotype distribution is extremely important for decision makers in public health services; in fact it allows conscious decision on vaccine choice for the population in all countries of the world.

On the other hand, molecular typing is an important tool also in studies on pneumococcal carriage. At present, individuation and typing of pneumococci in nasopharyngeal swabs is obtained by a single colony method [16-17]. Presently, using this method only a single serotype per patient can be found and multiple colonization, which is a very frequent condition, cannot be detected [18]. Therefore, there is the need for a method of detection of S. pneumoniae serogroup that has improved sensitivity in respect to current methods and that is cost effective.

The authors of the present invention have previously described that multiplex sequential PCR (MS-PCR) performed directly on clinical samples for pneumococcal serotyping has a sensitivity significantly higher than that of culture [3]. Even though the possibility of pneumococcal serotyping has been greatly improved by MS-PCR methods, serotyping could be further optimized by the use of Realtime-PCR known, at present, as one of the most specific and sensitive molecular method [19-20]. For this reason, the use and the combination of primers/probes obtained from specific genomic regions of Streptococcus pneumoniae, the set up of precise procedures for amplification with RT-PCR and the direct use of clinical samples has led to the identification of an innovative method compared to prior art.

### DESCRIPTION OF THE INVENTION

The present invention relates to a method in which Realtime PCR is used, directly on clinical samples, for typing multiple Pneumococcal serotypes, known to be the most frequent ones both in children and adults worldwide. The present invention has an improved sensitivity compared to prior art methods for identifying and serotyping *Streptococcus Pneumoniae* both in blood or otherwise body fluids and in nasopharyngeal swabs, as shown by examples described in the following sections.

The present invention has several advantages: 1) it allows the serotyping of non-viable bacteria, therefore samples which do not yield cultural growth can be serotyped; 2) it is more sensitive than multiplex sequential PCR because it uses realtime PCR amplification instead of endpoint amplification and identification on agarose gels.

Moreover, the technique described in the present invention has also the highest specificity when compared to methods already described in the state of the art:
1) when compared to multiplex sequential PCR, the use of specific and unique primers and probe sets allow the unique identification of a single serotype.
2) Standard PCR techniques are lengthy including the time for completion of the amplification steps, and the time for agarose gel electrophoresis; therefore the individuation of serotype is not possible in less than 4 hour. Moreover, since the MS-PCR techniques include multiple steps, if the serotype is not individuated in the first step, additional amplification/electrophoresis steps (3-4hours/each) may be needed rendering the whole method even more time expensive.
3) Cultural detection of bacterial strains can be sometimes difficult. By contrast, the present method allows the identification of a unique strain in DNA of isolates or bacterial DNA of clinical samples with no error.
3) the present method displays improved sensitivity in respect of molecular methods using RT-PCR based on degenerate primers which do not show good sensitivity and may lead to ambiguous results, as demonstrated by examples described in the following sections (28).

The present invention allows the use of clinical samples directly in Realtime PCR for *Streptococcus Pneumonia* serotyping. The sample taken from the human body may be any type of human biological tissue. Among these, blood, pleuric liquid and cefalorachidian liquor (CSF) are preferred. In the cases wherein an organ pathology (meningitis, pneumonia with pleuritis) is present, it is preferred that a sample be taken from the seat of infection (CSF or pleuric liquid). When these are not available and for the unlocalised forms (sepsis, bacteriaemia), whole blood is preferred, because the standard regulations for the sampling thereof and the displacement/freezing of the same are well known and practiced in the art, with equipment also known to the skilled in the art.

A further advantage of the method according to the present invention is that a sample of a biological tissue may be used, even if it has been sampled far away or however within a time interval greater than 1 day, while the maximum time for using the samples by culture is within one hour. Samples taken even 8 days earlier are still usable in the method according to the invention. The sample taken may optionally be frozen and defrosted at the time of analysis. Thus, with a method of freezing and defrosting of the sample, the presence and/or the serogroup and/or the serotype of a pathogen may be detected more than 8 days later and for an indefinite time. The only requirement of the method according to the invention is that the nucleic acid of the prokaryote remain intact and extractable and have not hydrolised or decomposed during the wait between the sampling and the beginning of the method.

The biological sample may be taken either in a test tube or absorbed as a spot on blotting paper, the blotting paper sheets being similar to those normally used for the Guthri test at birth, for the screening of hypothyroidism and phenylketonuria. An advantage of this method is that blood may be sampled also from a fingertip by capillary prick. Thus, the sampling may be done also at the patient's place of residence or in the doctor's office, since no sampling of venous blood is required. The sheet used for the spot (which is sterile prior to use), once used, must immediately be put away in a plastic sachet in order to avoid contaminations by environment germs and subsequently sent to the laboratory carrying out the test. In the contest of the method of the present invention, it is preferred to extract and use only DNA because RNA is labile and not preservable for a long time. Any method for extracting DNA from a sample of biological tissue known in the art may be used for the method of the invention. It is preferable to use a group of reagents or kits for extracting DNA comprising K protease, such as e.g. the QIAmp DNA mini kit, by Qiagen, Hilden, Germany. The use of K protease has shown, in comparative studies, a greater capability of retrieving bacterial DNA and said capability results in a greater sensitivity of the test.

The molecular method subject of the present invention can easily be used on stored biological samples, with no limitation of time. Therefore the method is suitable both for prospective and retrospective analysis allowing to give a complete epidemiological picture of invasive pneumococcal disease.

In the following section it is described how the presence of *Streptococcus Pneumoniae* DNA in biological samples and isolates can be evaluated by molecular methods subject of the present invention, preferably using primers and probes with at least one Real-Time Polymerase Chain Reaction (RT) or by methods previously described (MS-PCR). In a preferred embodiment, Primers and probes are designed based on *CpsA gene* (pneumococcal capsular polysaccharide synthesis gene, SEQ ID No. 64, Table 1). The example described in the section below shows the improvement obtained by the methods subject of the present invention when compared to methods previously described (MS-PCR).

Nonetheless, when a positive result is obtained in samples considered negative by other methods, such as MS-PCR or culture, it might be speculated that this is due to poor specificity of RT-PCR rather than to improved sensitivity. However amplification in RT-PCR at the same time of two different targets from two unrelated genes of *Streptococcus pneumoniae, lytA* (known to be extremely sensitive and specific) [21] and *CpsA* confirms the sample positivity.

Realtime PCR in pneumococcal serotyping offers additional chances also in the analysis of pharyngeal carrying. The method subject of the present invention being more sensitive than previously known methods, demonstrate multiple colonization can be found with a frequency that is higher than previously thought with methods known in the art [34].

This aspect has important implications in the study of pneumococcal carriage and serotype replacement. Actually, in carriage studies performed with culture the single colony method is commonly used to determine the serotype by the Quellung reaction. When colonies of multiple morphologies are present, selection by morphology adds some information, but nevertheless both methods underestimate the true rate of multiple carriage [16-18]. Actually, based on the single-colony cultural method, a single serotype per patient can be found and multiple colonization, which is a very frequent condition cannot be revealed [16] when we are interested in finding all circulating serotypes or when we want to draw conclusions on limited phenomena as initial serotype replacement could be, individuating multiple colonization is mandatory and therefore molecular methods should be used.

RT-PCR is a technique known in the art. Reaction amplification is signalled and, possibly, quantified by use of a fluorescent probe which generates a signal simultaneously with the amplification reaction The nucleic acid sequences resulting from the RT-PCR reaction are DNA sequences. The RT-PCR reaction may be carried out with any equipment or reagent known in the art. In a preferred embodiment of said aspect of the invention wherein the presence or absence of at least a pathogen is detected, use is made of primer and hydrolysis probe sequences, among which, e.g., the TaqMan probes may be used in a PCR amplification cycle reaction known as TaqMan reaction. Said TaqMan probe sequence is a fluorescent probe bonded by means of a nucleic acid sequence to a quencher. The nucleic acid sequence is DNA. The TaqMan probe sequence is hydrolised thanks to the polymerisation of Taq-polymerase (through the action of 3'-5' exonuclease of Taq polymerase). As a consequence, the fluorescent hydrolisis probe or TaqMan probe, without interferences from the quencher, emits fluorescence to signal that polymerization has occurred. TaqMan primers and probes used in the present invention may be built according to methods known in the art to build DNA sequences and methods to bind probes or quenchers thereto. In said aspect of the invention, the RT-PCR reaction may detect the presence of one or more sequences, and consequently, if the reagents are adequately selected, one or more pathogen serotypes at the same time. An advantage of the use of said aspect of the invention, which is the RT-PCR to detect the presence of one or more different sequences, is a reduction of times and/or costs to detect whether and which pathogen serotypes is present in the sample. RT-PCR enables the discrimination among said serotypes with the help of one ore more primer couples (the couple being forward primer and reverse primer for a specific sequence), designed to amplify genes or specific sequences. With the presence of a TaqMan probe, the amplification signals the presence in the sample of extracted nucleic acid. If every nucleic acid sequence is uniquely specific for a single pathogen among those mentioned above, the RT-PCR enables the signalling of their presence.

It is therefore an object of the invention a method for detecting a specific serogroup of *S*. *pneumonia* in a biological sample obtained from a subject, comprising the following steps:
a) extracting *S*. *pneumonia* DNAdirectly from the sample;
b) incubating the extracted DNA under conditions such as to enable the amplification of at least one serogroup specific target region comprised in SEQ ID No. 64 of the *CpsA* gene of *S*. *pneumoniae,* if present in the sample and
c) detecting the amplified product.

In a preferred embodiment of said aspect of the invention, the nucleic acid sequences are DNA because RNA is labile and does not keep for a long time and Taq DNA polymerase works better with DNA. In a more preferred embodiment, DNA sequences are genes or specific sequences of the serotypes. Still more preferably said genes or specific sequences of the serotypes are from CpsA gene.

Preferably the serogroup specific target region is comprised in the group of:
- nt 9301 to nt 9391 of SEQ ID No. 64 for serotype 1 of *S*. *Pneumoniae*
- nt 5341 to nt 5445 of SEQ ID No. 64 for serotype 3 of *S*. *Pneumoniae*
- nt 10561 to nt 10650 of SEQ ID No. 64 for serotype 4 of *S*. *Pneumoniae*
- nt 6268 to nt 6378 of SEQ ID No. 64 for serotype 5 of *S*. *Pneumoniae*
- nt 8761 to nt 8870 of SEQ ID No. 64 for serotype 6A/B of *S*. *Pneumoniae*
- nt 8614 to nt 8712 of SEQ ID No. 64 for serotype 7A/F of *S*. *Pneumoniae*
- nt 8581 to nt 8720 of SEQ ID No. 64 for serotype 8 of *S*. *Pneumoniae*
- nt 9181 to nt 9232 of SEQ ID No. 64 for serotype 9V/A of *S*. *Pneumoniae*
- nt 13081 to nt 13204 of SEQ ID No. 64 for serotype 10A/B of *S*. *Pneumoniae*
- nt 10561 to nt 10689 of SEQ ID No. 64 for serotype 12A/B/F of *S*. *Pneumoniae*
- nt 8761 to nt 8876 of SEQ ID No. 64 for serotype 14 of *S*. *Pneumoniae*
- nt 12181 to nt 12286 of SEQ ID No. 64 for serotype 15 of *S*. *Pneumonias*
- nt 9721 to nt 9833 of SEQ ID No. 64 for serotype 18B/C of *S*. *Pneumoniae*
- nt 14581 to nt 14683 of SEQ ID No. 64 for serotype 19 B/F of *S*. *Pneumoniae*
- nt 8701 to nt 8823 of SEQ ID No. 64 for serotype 19A of *S*. *Pneumoniae*
- nt 10081 to nt 10223 of SEQ ID No. 64 for serotype 20 of *S*. *Pneumoniae*
- nt 8641 to nt 8724 of SEQ ID No. 64 for serotype 22F/A of *S*. *Pneumoniae*
- nt 9841 to nt 9993 of SEQ ID No. 64 for serotype 23F of *S*. *Pneumoniae*
- nt 8161 to nt 8260 of SEQ ID No. 64 for serotype 33 A/F of *S*. *Pneumoniae*
- nt 8401 to nt 8679 of SEQ ID No. 64 for serotype 35B of *S*. *Pneumoniae*
- nt 12181 to nt 12267 of SEQ ID No. 64 for serotype 38 of *S. Pneumoniae*

Preferably, the steps of amplification and detection occur by RT-PCR.

It is a further object of the invention a kit for detecting a specific serogroup of *S*. *pneumonia* in a biological sample obtained from a subject comprising at least one primer oligonucleotide capable of amplifying at least one serogroup specific target region indicated above , at least one probe oligonucleotide allowing to detect at least one amplified serogroup specific target region indicated above and control target regions.

Preferably primer and probe oligonucleotides are selected from the group of:
- SEQ ID NO. 1 and 2 as forward and reverse primer for serotype 1 and SEQ ID NO. 3 as TaqMan probe sequence for serotype 1
- SEQ ID NO. 4 and 5 as forward and reverse primer for serotype 3 and SEQ ID NO. 6 as TaqMan probe sequence for serotype 3
- SEQ ID NO. 7 and 8 as forward and reverse primer for serotype 4 and SEQ ID NO. 9 as TaqMan probe sequence for serotype 4
- SEQ ID NO. 10 and 11 as forward and reverse primer for serotype 5 and SEQ ID NO. 12 as TaqMan probe sequence for serotype 5
- SEQ ID NO. 13 and 14 as forward and reverse primer for serotype 6 A/B and SEQ ID NO. 15 as TaqMan probe sequence for serotype 6A/B
- SEQ ID NO. 16 and 17 as forward and reverse primer for serotype 7 A/F and SEQ ID NO. 18 as TaqMan probe sequence for serotype 7F
- SEQ ID NO. 19 and 20 as forward and reverse primer for serotype 8 and SEQ ID NO. 21 as TaqMan probe sequence for serotype 8
- SEQ ID NO. 22 and 23 as forward and reverse primer for serotype 9V/A and SEQ ID NO. 24 as TaqMan probe sequence for serotype 9V/A
- SEQ ID NO. 25 and 26 as forward and reverse primer for serotype 10A/B and SEQ ID NO. 27 as TaqMan probe sequence for serotype 10AF
- SEQ ID NO. 28 and 29 as forward and reverse primer for serotype 12A/B/F and SEQ ID NO. 30 as TaqMan probe sequence for serotype 12ABF
- SEQ ID NO. 31 and 32 as forward and reverse primer for serotype 14 and SEQ ID NO. 33 as TaqMan probe sequence for serotype 14
- SEQ ID NO. 34 and 35 as forward and reverse primer for serotype 15 and SEQ ID NO. 36 as TaqMan probe sequence for serotype 15
- SEQ ID NO. 37 and 38 as forward and reverse primer for serotype 18B/C and SEQ ID NO. 39 as TaqMan probe sequence for serotype 18B/C
- SEQ ID NO. 40 and 41 as forward and reverse primer for serotype 19A and SEQ ID NO. 42 as TaqMan probe sequence for serotype 19A
- SEQ ID NO. 43 and 44 as forward and reverse primer for serotype 19B/F and SEQ ID NO. 45 as TaqMan probe sequence for serotype 19B/F
- SEQ ID NO. 46 and 47 as forward and reverse primer for serotype 20 and SEQ ID NO. 48 as TaqMan probe sequence for serotype 20
- SEQ ID NO. 49 and 50 as forward and reverse primer for serotype 22F/A and SEQ ID NO. 51 as TaqMan probe sequence for serotype 22F/A
- SEQ ID NO. 52 and 53 as forward and reverse primer for serotype 23F and SEQ ID NO. 54 as TaqMan probe sequence for serotype 23F
- SEQ ID NO. 55 and 56 as forward and reverse primer for serotype 33 A/F and SEQ ID NO. 57 as TaqMan probe sequence for serotype 33 A/F
- SEQ ID NO. 58 and 59 as forward and reverse primer for serotype 35B and SEQ ID NO. 60 as TaqMan probe sequence for serotype 35B
- SEQ ID NO. 61 and 62 as forward and reverse primer for serotype 38 and SEQ ID NO. 63 as TaqMan probe sequence for serotype 38
wherein the primers for the control region are obtained from lytA gene and are SEQ ID 1 and SEQ ID 2, or SEQ ID 7 and SEQ ID 8.

Preferably, primer oligonucleotides are grouped in a plurality of reaction environments. Each group may contain 2 primer/probe sets. Any combination of primer/probe sets specific for different serotypes is accepted.

The RT-PCR reaction is carried out for n amplification cycles, preferably more than 40, even more preferably from 43 to 45 cycles, even more preferably 45 cycles. The reliability of the diagnostic outcome from this aspect of the invention is improved based on the number of RT-PCR cycles and 45 is the best value. An amplification of more than 45 cycles does not improve the reliability of the diagnostic outcome. The diagnostic outcome is compared with the threshold cycle (CT). The CT is the cycle wherein the fluorescence signals emitted are neatly measurable and statistically valid relative to the background noise. If at CT a significantly higher fluorescence and statistically meaningful is found, there may be determined, based on the frequency of the fluorescence emitted and on how the TaqMan fluorescent probes have been prepared in the TaqMan probes, which sequence, and therefore which serotype, is present in the sample and which is not. If no spontaneous fluorescence (i.e. different from the background noise) is measured, one may infer that the sequences specific for the serotype are not present in the DNA extracted from the sample taken.

According to said aspect of the invention, if the presence of said genes at the end of said RT-PCR reaction is detected or not detected, it may be concluded that said serotype is or is not present in the sample taken from the host. Embodiments of said aspect of the invention are reported in even greater detail in the example. An advantage of the present method over the methods of the known art is that it allows the use of nucleic acid, preferably DNA extracted from a sample of biological tissue.

### EXAMPLE

### Streptococcus Pneumoniae isolates

Eight bacterial strains of *Streptococcus Pneumoniae* were obtained from ATCC (respectively) ATCC 6301 for 1, ATCC 6304 for 4, ATCC 6305 for 5, ATCC 10357 for 19A, ATCC 6323 for 23F, ATCC 6326 for 6B, ATCC 10368 for 9V, ATCC BAA-659 for 6A), 38 well characterized S. pneumoniae isolates from eighteen serotypes (1, 3, 4, 5, 6A, 6B, 7F, 8, 9V, 12F, 14, 15B/C, 18C, 19A, 19F, 22F, 23F,35B) which had been isolated from blood or CSF or throat swab samples and stored in germ bank at the Microbiology Laboratory of the Anna Meyer Children Hospital were used for molecular serotyping.

### Biological samples

Sixty-nine biological samples (27 blood samples, 4 cerebrospinal fluid (CSF) samples, 4 pleural fluids and 34 pharyngeal swabs), found positive for *Streptococcus Pneumoniae* by Realtime PCR on *lytA* gene as previously described [3] and obtained from children with a clinical suspicion of invasive pneumococcal infection were tested for *CpsA* by standard PCR and agarose gel electrophoresis as described in following paragraphs; samples positive for both *lytA* and *CpsA* were included in serotyping analyses both by multiplex-sequential PCR (MS-PCR) and Realtime-PCR.

### Bacterial DNA extraction

Bacterial genomic DNA was extracted from bacterial strains or biological samples using QIAmp DNeasy Blood & Tissue kit (Qiagen, Hilden, Germany) with protocol for blood and body fluids, according to the manufacturer's instructions for extraction of a 200 µL sample.

### Molecular diagnosis di Streptococcus pneumoniae

The presence of *Streptococcus Pneumoniae* DNA in biological samples and isolates was evaluated by molecular methods as previously described [3]. Briefly, primers and probes within the autolysin gene *lytA* [21] were used for RT amplification at a final concentration of 300 nM for primers, and 50 nM for JOE-labeled probes. The cycle threshold (C*_{T}*) value is the PCR cycle number (out of 45) at which the measured fluorescent signal exceeds a calculated background threshold identifying amplification of the target sequence. If no increase in fluorescent signal is observed after 45 cycles, the sample is assumed to be negative. The presence of *Streptococcus Pneumonia* was furtherly confirmed by the amplification of *CpsA* gene in multiplex sequential PCR as previously described [3-4] and briefly described in the next paragraph.

### PCR serotyping by Multiplex sequential PCR and standard cultural methods

Molecular serotyping by sequential multiplex PCR was performed both on bacterial isolates and directly on clinical samples. All the RT-PCR positive samples were included in the analysis. Briefly, thirty-one primer couples [3] were grouped into nine multiplex reactions. *CpsA* (pneumococcal capsular polysaccharide synthesis gene) primers were included in all the reaction mix as a confirmatory test [3, 22]. Amplification was performed in a Perkin-Elmer GeneAmp PCR system 2720 (Applied Biosystems, Foster City, CA, USA) under the following conditions: 95°C for 15 minutes followed by 35 amplification cycles of 94°C for 30 seconds, 54°C for 90 seconds, and 72°C for 60 seconds. A final hold was performed at 72°C for 10 minutes. The PCR products were analyzed by gel electrophoresis on 2% NuSieve agarose gels (Cambrex Bio Science, Inc., Rockland, ME) in 1x TAE buffer.

Cultural serotyping was performed using specific pneumococcal antisera on bacterial isolates (Biogenetics Srl, Padova, Italy) following manufacturer instructions.

### PCR serotyping by Realtime PCR

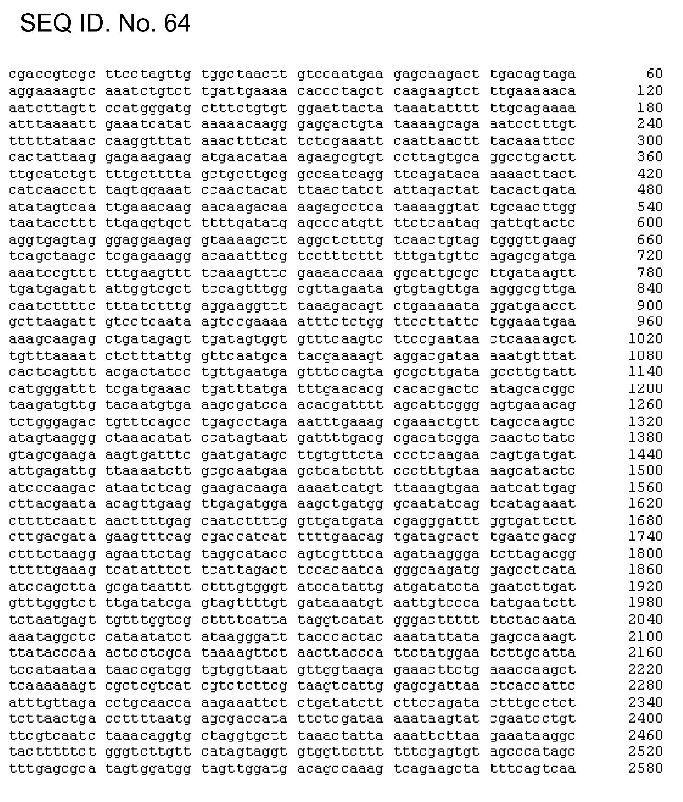

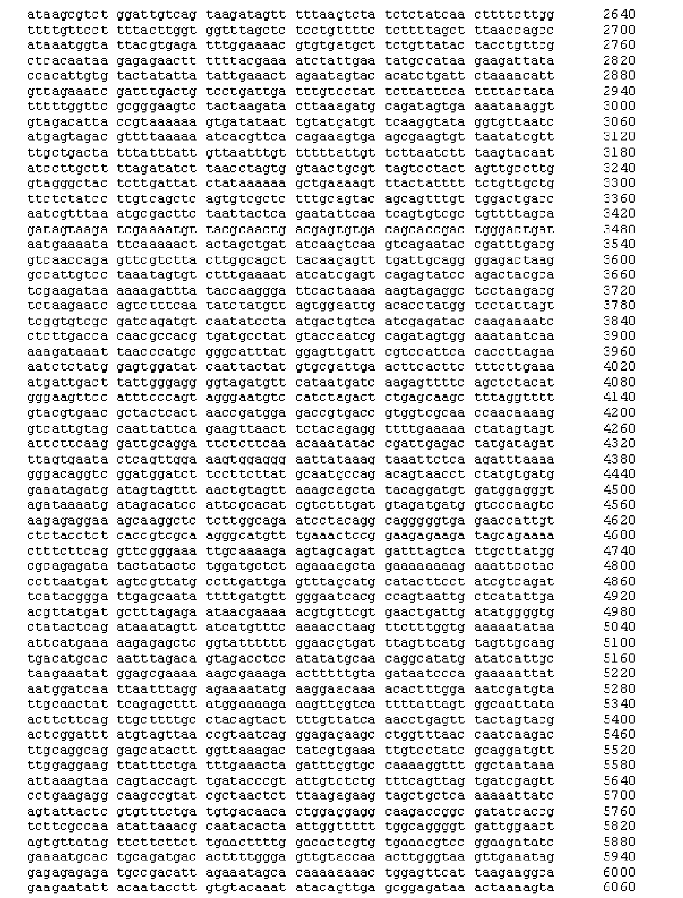

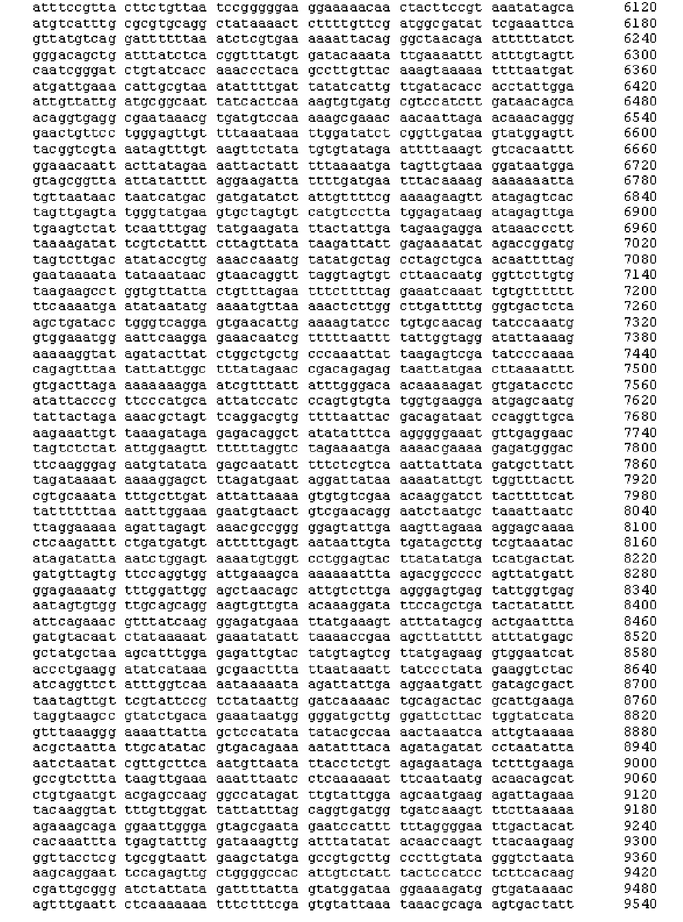

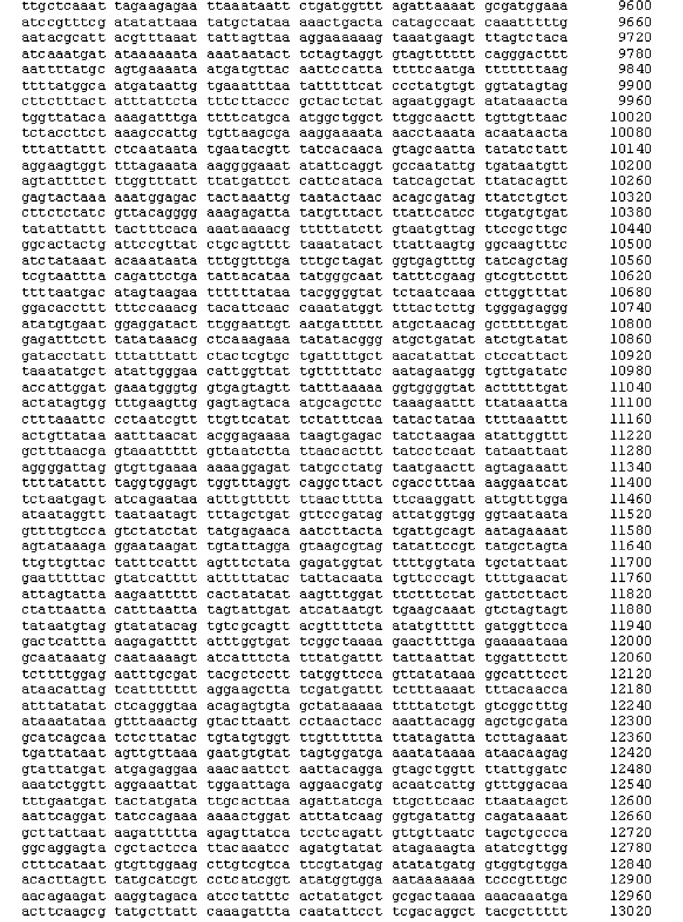

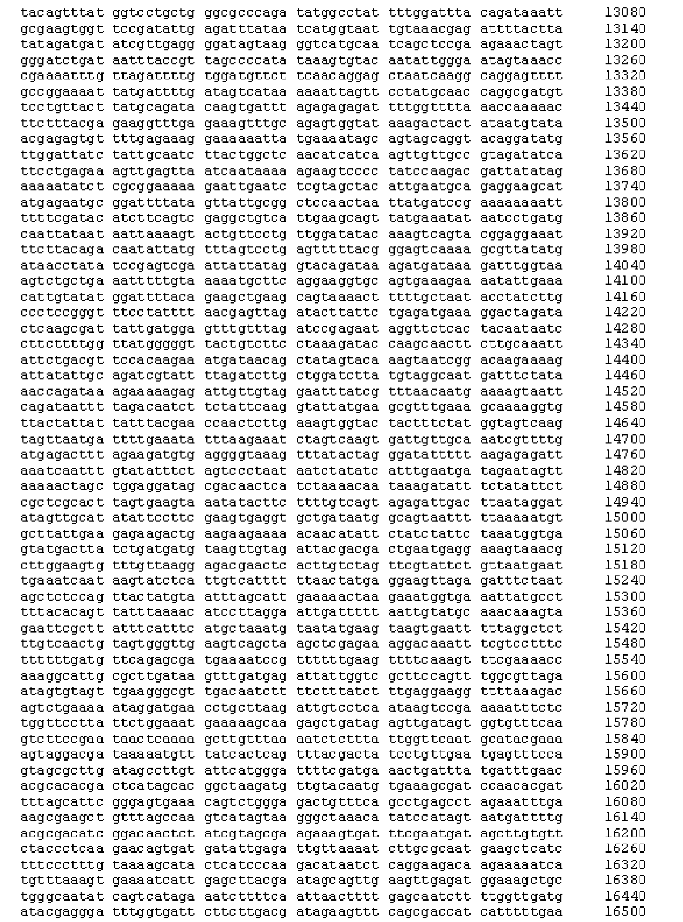

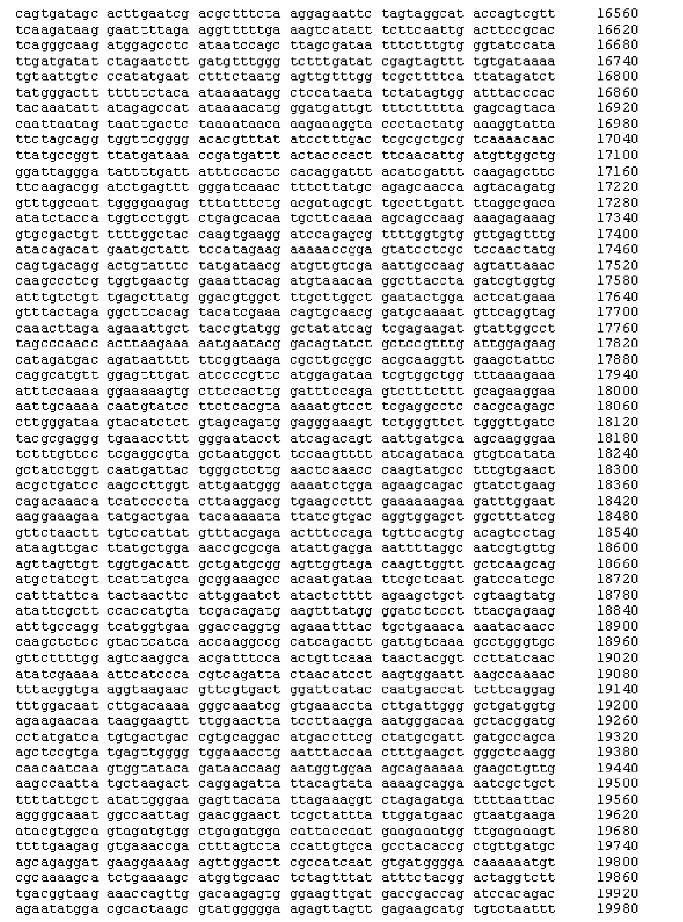

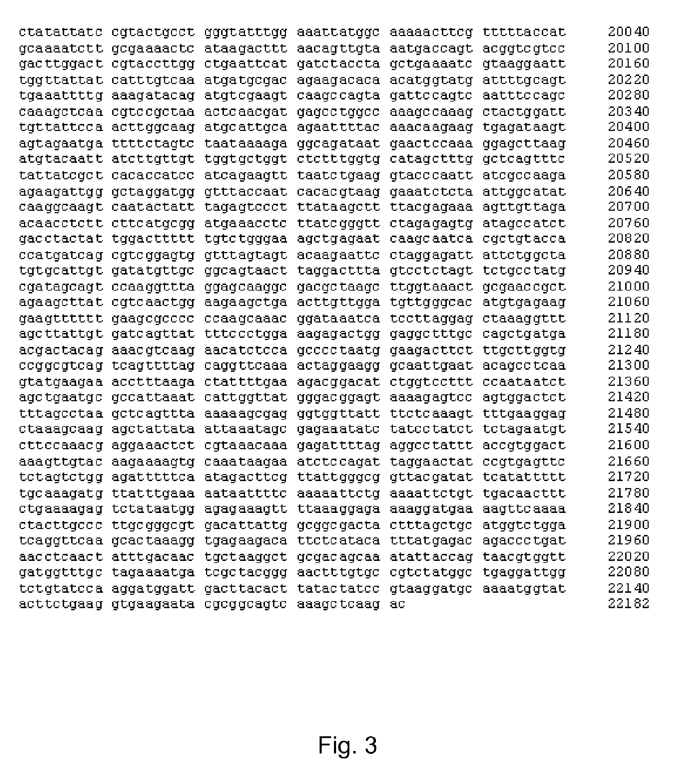

Primers and probes (Table 1) were designed using the ABI Primer Express Software Package based on previously published *CpsA gene* (SEQ ID. no. 64, Fig. 3). The RT amplification was performed in 25 µL reaction volumes containing 2x TaqMan Universal Master Mix (Applied Biosystem, Foster City, CA, USA); primers and JOE labeled probes were used at a concentration of 400nM; FAM labeled probes at a concentration of 200 nM. Six µl of DNA extract were used for each reaction. All reactions were performed in triplicate. A negative control (no-template) and a positive control were included in every run. DNA was amplified in an ABI 7500 sequence detection system (Applied Biosystem, Foster City, CA, USA) using, for all the primers couples, the same cycling parameters as follows: 50° for 2 min for UNG digestion, 95°C for 10 min followed by 45 cycles of a two-stage temperature profile of 95°C for 15 sec and 60°C for 1 min.

As for molecular diagnosis of IPD (invasive pneumococcal didease) by realtime PCR, if no increase in fluorescent signal was observed after 45 cycles, the sample was assumed to be negative and was reported as non-typeable.

### Pneumococcal serotype classification and correlation with the existing conjugate vaccine

As previously described [23], pneumococcal serotypes were classified as "PCV-7 serotypes" if they were included in the conjugate vaccine (4,6B,9V,14,18C,19F or 23F). They were classified as "PCV-7 related serotypes" if they were from the same serogroups and were assumed or known to be cross-reactive with PCV7 serotypes (6A,9A,9L,9N,18A,18B,18F,19B,19C,23A,23B); all the other, including 19A, known to be non cross-reactive with 19F, were classified as "non-vaccine serotypes" [23].

### Statistical analysis

Data were evaluated to determine how many samples were positive by each test, as well as those positive by two tests and those positive by only one test. Agreement between the two tests was assessed by Cohen's kappa statistic, with values of 0.00 to 0.20 indicating poor agreement, 0.21 to 0.40 indicating fair agreement, 0.41 to 0.60 indicating moderate agreement, 0.61 to 0.80 indicating good agreement, and 0.81 to 1.00 indicating excellent agreement. Marginal homogeneity of the two tests was assessed by McNemar's test. Results were expressed as mean levels and standard deviations. All continuous variables were expressed as mean ± SD. Fisher exact test and Pearson's chi-square test were used when appropriate.

### External Quality Control

An External Quality Control for Real-time PCR and standard PCR is regularly carried out with excellent results in the Laboratory of Immunology where all the molecular test were performed.

### RESULTS

During the set up of the method subject of the present invention, only 15 primer/probe set, representing the most frequent *Streptococcus Pneumoniae* serotypes in western world, were initially included. With such group of primer/probe it was possible to identify and serotype 92% of IPD (invasive pneumococcal disease) cases. A group of 6 additional primer/probe was then added giving a 4% increase in serotype identification. Therefore, it was found that in a preferred embodiment, the present invention uses a panel of primer/probe including 21 serotypes that allows to achieve, at present, pneumococcal serotyping in over 94% cases in Italy and likely in most western countries where the distribution is similar. In details, using the primer/probe sets described in the present invention the highest efficiency and the best cost/benefit ratio are obtained. In fact, the optimized combination gives the highest frequency of serotyping results with the less expense. Moreover, the method of the present invention allows to include primers/probe for additional serotypes, therefore it can be adapted to possible epidemiological changes and serotype shifts over the years and in different parts of the world.

The amplified sequences detected with the primer/probe sets here described can be considered as molecular markers for invasive pneumococcal infections due to different serotypes. Each amplified sequence is to be considered, therefore as a specific marker for a unique infection.

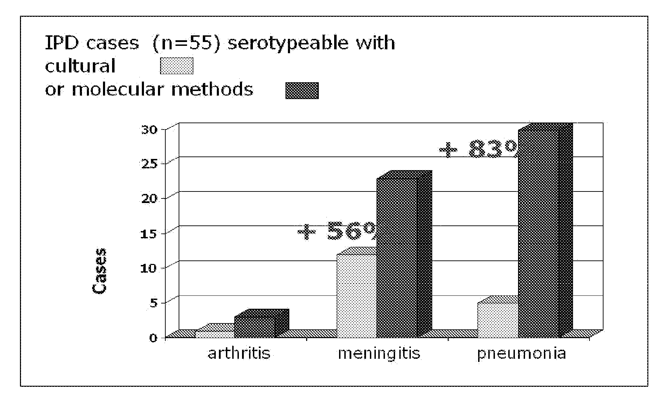

In conclusion, using the present invention's method, based on Realtime PCR in a clinical setting, it is possible to achieve diagnosis and serotyping of invasive pneumococcal infection in cases for which the diagnosis was not possible with other methods used before. As from examples given in the section below, 83% pneumonia cases diagnosed and serotyped by Realtime PCR would not have been serotyped using only cultural methods, similarly 56% meningitis would not have been serotyped (Fig.1).

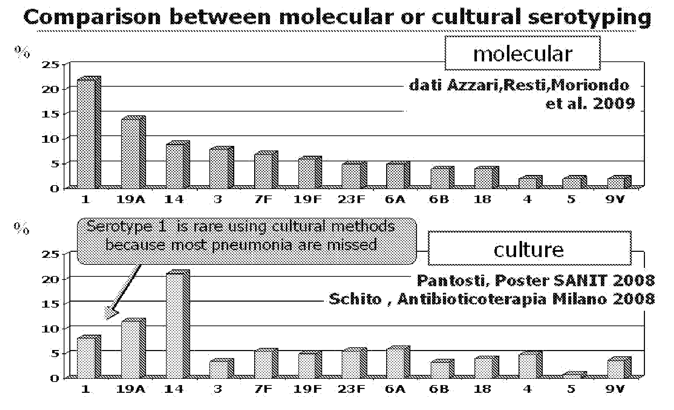

Moreover, serotype distribution as evidenced by Realtime PCR appears different from what is previously known. Since present cultural methods fail to detect most cases of bacteremic pneumococcal pneumonia, the frequency of serotype 1 detected using such method is significantly lower than that detected by Realtime PCR (Fig.2).

### Molecular diagnosis of Streptococcus pneumoniae

### Isolates

All the pneumococcal isolates (either obtained from ATCC or obtained from the microbiology lab) resulted positive with both MS-PCR (CpsA gene) and RT PCR (lytA gene).

### Biological samples

Positivity for both *CpsA* in MS-PCR and *lytA* in RT-PCR was found in 67/69 (97.1 %) samples. The two samples (blood) negative for *CpsA* in MS-PCR had a RT-PCR C*_{T}* respectively of 38 and 39. Only the 67 samples positive for both *lytA* gene in RT-PCR and *CpsA* gene in MS-PCR were included in the study.

### Serotyping of Streptococcus Pneumoniae isolates

The eight isolates obtained from ATCC were serotyped by both molecular methods and were consistent with ATCC serotype.

All the 38 *Streptococcus Pneumoniae* isolates serotyped by the two molecular methods showed a complete concordance with serologic typing (serotype 3:7 isolates; serotypes 1, 4, 5, 6A: 3 isolates/each; serotypes 6B,7F, 8, 9V, 12F, 14: 2 isolates/each; serotypes 15B/C, 18C, 19A, 19F, 22F, 23F, 35B 1 isolate/each). No cross-reaction between serotypes was found for any primer/probe set.

### Serotyping of Streptococcus Pneumoniae from biological samples

Among the 67 biological samples found positive for Streptococcus pneumoniae, 43/67 (64.2%; 16/25 blood samples; 4/4 CSF; 4/4 pleural fluid, 19/34 swabs) could be serotyped by MS-PCR, while 61/67 (23/25 whole blood, 4/4 CSF, 4/4 pleural fluid, 30/34 pharyngeal swabs 91.0 %, p=0.001) could be serotyped by RT-PCR (K cohen:0.3; McNemars's p<0.001). The 21 biological samples that could be serotyped only by RT-PCR (7/25 blood samples, 12/34 pharyngeal swabs) had a C*_{T}* (threshold cycle) ≥30 (median 34.5; SD 2.4; range 30-38 with no difference between blood samples and swabs). All CSF and pleural fluid samples could be serotyped with both methods. One sample (pharyngeal swab) that could be serotyped only by MS-PCR was a serotypes 6C/D, primers of which were not available for RT-PCR. Five samples (2 blood samples, 3 pharyngeal swabs) could not be serotyped with any method.

When only samples obtained from otherwise sterile fluids (blood, CSF, pleural fluid) were analysed, MS-PCR allowed serotyping in 24/33 (72.7%) samples (16 WB, 4 CSF, 4 PF obtained from 25 patients) and RT-PCR in 31/33 (93.9%) samples (23 WB, 4 CSF, 4PF) (p=0.047; 95% CL 0.03-0.98). Serotypes found with the two molecular methods are shown in Table 2.

**Table 2- Comparison between Multiplex sequential PCR (MS-PCR) and Realtime PCR (RT-PCR) in evaluating serotype distribution in sterile fluids from 25 patients with pneumonia (P) or meningitis (M) .**

| | **Disease** | **MS-PCR** | **RT- PCR** |
|---|---|---|---|
| 1 | P | 19A | 19A |
| 2 | P | 19A | 19A |
| 3 | P | 1 | 1 |
| 4 | S | 22A/F | 22 A/F |
| 5 | P | 1 | 1 |
| 6 | M | NT | NT |
| 7 | M | NT | NT |
| 8 | M | NT | 18 |
| 9 | M | 19A | 19A |
| 10 | P | NT | 19F/B |
| 11 | P | NT | 19A |
| 12 | M | 7A/F | 7 A/F |
| 13 | P | NT | 1 |
| 14 | P | NT | 1 |
| 15 | P | NT | 1 |
| 16 | P | NT | 19A |
| 17 | M | 23F | 23F |
| 18 | M | 13F | 23F |
| 19 | P | 6A | 6A |
| 20 | M | 14 | 14 |
| 21 | M | 6B | 6B |
| 22 | M | 8 | 8 |
| 23 | P | 19 A | 19A |
| 24 | M | 10 A/B | 10 A/B |
| 25 | M | 23F | 23F |

Multiple colonization by different pneumococcal serotypes was demonstrated in 20/30 (66.7%) pharyngeal swabs typed by RT-PCR, and in 3/19 (15.8%) swabs typed by MS-PCR (p=0.002; 95%CL=2.02-44.13). Realtime PCR demonstrated 1 single serotype in 10/30 (33.3%), 2 different serotypes in 13/30 (43.3%), 3 different serotypes in 6/30 (20.0%) and 4 different serotypes in 1/30 (3.3%); MS-PCR demonstrated 2 different serotypes in 3/19 (15.8%), while in 16/19 samples (84.2%) only one serotype was found (Table 3).

**Table 3 - Multiple pharyngeal colonization shown by Multiplex-sequential PCR (MS) or Real-time PCR (RT) in 34 pharyngeal swabs**

| Sample ID | Method | Serotypes identified | | | |
|---|---|---|---|---|---|
| | | I serotype | II serotype | III serotype | IV serotype |
| 1 | MS | 22 A/F | | | |
| | RT | 22A/F | 19A | 19F | |
| 2 | MS | NT | | | |
| | RT | NT | | | |
| 3 | MS | 19F | | | |
| | RT | 19F | 5 | | |
| 4 | MS | NT | | | |
| | RT | 4 | 5 | 19F/B | |
| 5 | MS | 6C/D | | | |
| | RT | NT | | | |
| 6 | MS | 5 | | | |
| | RT | 5 | 6A/B | | |
| 7 | MS | 20 | | | |
| | RT | 20 | | | |
| 8 | MS | NT | | | |
| | RT | NT | | | |
| 9 | MS | 9V/A | | | |
| | RT | 9V/A | 5 | | |
| 10 | MS | 14 | | | |
| | RT | 14 | | | |
| 11 | MS | 5 | | | |
| | RT | 5 | 9V/A | 19F/B | |
| 12 | MS | NT | | | |
| | RT | 1 | 19A | | |
| 13 | MS | 22 A/F | | | |
| | RT | 22 A/F | 19A | | |
| 14 | MS | NT | | | |
| | RT | 5 | 19F/B | | |
| 15 | MS | 5 | | | |
| | RT | 5 | 6A/B | | |
| 16 | MS | 5 | | | |
| | RT | 5 | 4 | | |
| 17 | MS | 18 | | | |
| | RT | 18 | 19F/B | 19A | |
| 18 | MS | NT | | | |
| | RT | 1 | 19F/B | | |
| 19 | MS | 1 | | | |
| | RT | 1 | | | |
| 20 | MS | 3 | | | |
| | RT | 3 | 5 | 19F/B | |
| 21 | MS | NT | | | |
| | RT | 1 | | | |
| 22 | MS | NT | | | |
| | RT | 19F/B | | | |
| 23 | MS | NT | | | |
| | RT | 5 | 3 | | |
| 24 | MS | NT | | | |
| | RT | 19F/B | | | |
| 25 | MS | NT | | | |
| | RT | 19F/B | | | |
| 26 | MS | 20 | 5 | | |
| | RT | 20 | 5 | | |
| 27 | MS | NT | | | |
| | RT | NT | | | |
| 28 | MS | 3 | 5 | | |
| | RT | 3 | 5 | | |
| 29 | MS | 19F/B | | | |
| | RT | 19F/B | 18 | | |
| 30 | MS | NT | | | |
| | RT | 1 | | | |
| 31 | MS | 5 | | | |
| | RT | 5 | 7A/F | 18 | 19F/B |
| 32 | MS | 23F | 18 | | |
| | RT | 23F | 18 | 14 | |
| 33 | MS | NT | | | |
| | RT | 19F/B | | | |
| 34 | MS | NT | | | |
| | RT | 18 | | | |
| Total serotypes in MS-PCR | | 22 | | | |
| Mean value per pharyngeal swab in 1.2 MS-PCR (range) | | serotypes (1-2) | | | |
| Total serotypes in RT-PCR | | 58 | | | |
| Mean value per pharyngeal swab 1.9 In RT-PCR (range) | | serotypes (range 1-4) | | | |

The mean number of serotypes found in each nasopharyngeal swab was 1.2 (range 1-2) for MS-PCR and 1.9 (range 1-4) for RT-PCR (p=0.0006; 95% CL= 0.25-0.75). Multiple colonization was never found in blood or CSF or pleural fluid.

Multiplex sequential PCR allows pneumococcal serotyping in about two/third cases (64.2%) when used directly on clinical samples (blood, CSF, pleural fluid or nasopharingeal swabs) while Realtime PCR allows pneumococcal serotyping in over 90% cases using a panel of 15 primer/probe sets, with a good concordance between the two tests. However infections due to different serotypes could be diagnosed using additional primer/probe sets. This flexibility is important to make the method suitable all over the world. A starting panel of 30 or more serotypes could be considered, but it would not be economical; in fact a panel of 15 primer/probe sets cover over 90% serotypes while a panel of 30 primer/probe sets adds little more than an additional 5%. However, while in Italy and in other western countries the 15-serotype-panel used in the present research is very efficient in pneumococcal serotyping, individuating with low cost the serotypes in over 90% cases, other serotypes could be more frequent in other parts of the world [29] so requiring different primer/probe sets. Moreover distribution of pneumococcal serotypes may change over years, both as a consequence of immune pressure [30] and of secular trend [31-33]. The present techniques allows to include other primer/probe sets whenever needed without changing laboratory protocols; other primer/probe sets can be easily added or can substitute one or more of the primers included in the present invention Other primer/probe sets can be included in a formulation of the test suitable for other countries in the world.

### REFERENCES

1.Corless CE, et al., J Clin Microbiol 2001; 39: 1553-1558.
2.Pai R., et al., J Clin Microbiol 2006, 44, 124-31
3.Azzari C, et al., J Med Microbiol 2008, 57:1205-1212.
4.Resti M, et al., Clin Therapeutics, 2009, 31: 1266-73.
5.Tarallo L, et al., Vaccine. 2006;24(47-48):6938-43
6.Bridy-Pappas AE, et al., Pharmacotherapy 2005;25:1193-212.
7.World Health Organization (WHO). Wkly Epidemiol Rec 2007; 82:93-104
8.Garau J, et al., Clin Infect Dis. 2007;45(1):52-4.
9.Arai, S., et al., Microbiol Immunol, 2001, 45, 159-162.
10.Lalitha, M. K., et al., J Clin Microbiol 1999 37, 263-265.
11.Barker, J. H. et al., J Clin Microbiol 199, 37, 4039-4041.
12.Heidelberger, M. (1983). Infect Immun 41, 1234-1244.
13.Henrichsen, J. (1999).. Am J Med 107, 50S-54S.
14.Kumar, A., Mariappuram, J. & Kim, C. H. (1985). Diagn Microbiol Infect Dis 3, 509-514.
15. Whitney CG, et al. N Engl J Med 2003;348:1737-46.
16. Charalambous BM, et al., J Clin Microbiol. 2008 May 21.
17.Hare KM, et al., Pediatr Infect Dis J. 2008;27(2):178-80.
18.Azzari C, et al., Clin Infect Dis, 2008; 47:997-999.
19.Heid CA, et al., Genome Res. 1996 Oct;6(10):986-94.
20.Mackay IM. Clin Microbiol Infect 2004; 10:190-212.
21. Carvalho MG, et al. 2007, J Clin Microbiol 45:2460-2466
22. Morrison KE, et al., 2000 J Clin Microbiol 38:434-437
23. Hsu HE, et al., N Engl J Med. 2009 Jan 15;360(3):244-56.
24.Sandgren A, et al., 2004 J Infect Dis 189:785-796
25.Martell M, et al., J Clin Microbiol. 1999 Feb;37(2):327-32.;
26.Bièche I, et al., Int J Cancer. 1998 Nov 23;78(5):661-6.
27.Lallemand F, et al., J Clin Microbiol. 2000 Apr;38(4):1404-8).
28. Tarragó D, et al., Clin Microbiol Infect. 2008 Sep;14(9):828-34.
29. Scott JA, et al., 1996 Clin Infect Dis 22:973-981
30. McEllistrem MC, et al., J Infect Dis 2003; 188:1679-84
31.Byington CL, et al., Clinical Infectious Diseases 2005; 41:21-9
32.Finland M, et al., J Clin Microbiol 1977; 5 :154-166
33.Butler JC, et al., J Infect Dis, 1995; 171:885-889.
34. Rubin LG, Rizvi A. et al., J. Med Microbiol 2004; 53: 595-602

## Claims

1. Method for detecting a specific serogroup of *S*. *pneumonia* in a biological sample obtained from a subject, comprising the following steps:
a) extracting *S*. *pneumoniae* DNAdirectly from the sample;
b) incubating the extracted DNA under conditions such as to enable the amplification of at least one serogroup specific target region comprised in SEQ ID No. 64 of the *CpsA* gene of *S*. *pneumoniae,* if present in the sample and
c) detecting the amplified product.

2. The method according to claim 1 wherein the serogroup specific target region is comprised in the group of:
- nt 9301 to nt 9391 of SEQ ID No. 64 for serotype 1 of *S*. *Pneumoniae*
- nt 5341 to nt 5445 of SEQ ID No. 64 for serotype 3 of *S*. *Pneumoniae*
- nt 10561 to nt 10650 of SEQ ID No. 64 for serotype 4 of *S*. *Pneumoniae*
- nt 6268 to nt 6378 of SEQ ID No. 64 for serotype 5 of *S*. *Pneumoniae*
- nt 8761 to nt 8870 of SEQ ID No. 64 for serotype 6A/B of *S*. *Pneumoniae*
- nt 8941 to nt 8712 of SEQ ID No. 64 for serotype 7A/F of *S*. *Pneumoniae*
- nt 8581 to nt 8720 of SEQ ID No. 64 for serotype 8 of *S*. *Pneumoniae*
- nt 9181 to nt 9232 of SEQ ID No. 64 for serotype 9V/A of *S*. *Pneumoniae*
- nt 13081 to nt 13204 of SEQ ID No. 64 for serotype 10A/B of *S*. *Pneumoniae*
- nt 10561 to nt 10689 of SEQ ID No. 64 for serotype 12A/B/F of *S*. *Pneumoniae*
- nt 8761 to nt 8876 of SEQ ID No. 64 for serotype 14 of *S*. *Pneumoniae*
- nt 12181 to nt 12286 of SEQ ID No. 64 for serotype 15 of *S*. *Pneumoniae*
- nt 9721 to nt 9833 of SEQ ID No. 64 for serotype 18B/C of *S*. *Pneumoniae*
- nt 14581 to nt 14683 of SEQ ID No. 64 for serotype 19 B/F of *S*. *Pneumoniae*
- nt 8701 to nt 8823 of SEQ ID No. 64 for serotype 19A of *S*. *Pneumoniae*
- nt 10081 to nt 10223 of SEQ ID No. 64 for serotype 20 of *S*. *Pneumoniae*
- nt 8641 to nt 8724 of SEQ ID No. 64 for serotype 22F/A of *S*. *Pneumoniae*
- nt 9841 to nt 9993 of SEQ ID No. 64 for serotype 23F of *S*. *Pneumoniae*
- nt 8161 to nt 8260 of SEQ ID No. 64 for serotype 33 A/F of *S*. *Pneumoniae*
- nt 8401 to nt 8679 of SEQ ID No. 64 for serotype 35B of *S*. *Pneumoniae* or
- nt 12181 to nt 12267 of SEQ ID No. 64 for serotype 38 of *S*. *Pneumoniae*

3. The method according to any one of previous claims wherein the steps of amplification and detection occur by RT-PCR.

4. Kit for detecting a specific serogroup of *S. pneumoniae* in a biological sample obtained from a subject comprising at least one primer oligonucleotide capable of amplifying at least one serogroup specific target region indicated in claims 1 to 2 , at least one probe oligonucleotide allowing to detect at least one amplified serogroup specific target region indicated in claims 1 to 2 and control target regions.

5. Kit according to claim 4 wherein primer and probe oligonucleotides are selected from the group of:
- SEQ ID NO. 1 and 2 as forward and reverse primer for serotype 1 and SEQ ID NO. 3 as TaqMan probe sequence for serotype 1;
- SEQ ID NO. 4 and 5 as forward and reverse primer for serotype 3 and SEQ ID NO. 6 as TaqMan probe sequence for serotype 3;
- SEQ ID NO. 7 and 8 as forward and reverse primer for serotype 4 and SEQ ID NO. 9 as TaqMan probe sequence for serotype 4;
- SEQ ID NO. 10 and 11 as forward and reverse primer for serotype 5 and SEQ ID NO. 12 as TaqMan probe sequence for serotype 5;
- SEQ ID NO. 13 and 14 as forward and reverse primer for serotype 6 A/B and SEQ ID NO. 15 as TaqMan probe sequence for serotype 6A/B;
- SEQ ID NO. 16 and 17 as forward and reverse primer for serotype 7 A/F and SEQ ID NO. 18 as TaqMan probe sequence for serotype 7F;
- SEQ ID NO. 19 and 20 as forward and reverse primer for serotype 8 and SEQ ID NO. 21 as TaqMan probe sequence for serotype 8;
- SEQ ID NO. 22 and 23 as forward and reverse primer for serotype 9V/A and SEQ ID NO. 24 as TaqMan probe sequence for serotype 9V/A;
- SEQ ID NO. 25 and 26 as forward and reverse primer for serotype 10A/B and SEQ ID NO. 27 as TaqMan probe sequence for serotype 10AF;
- SEQ ID NO. 28 and 29 as forward and reverse primer for serotype 12A/B/F and SEQ ID NO. 30 as TaqMan probe sequence for serotype 12ABF;
- SEQ ID NO. 31 and 32 as forward and reverse primer for serotype 14 and SEQ ID NO. 33 as TaqMan probe sequence for serotype 14;
- SEQ ID NO. 34 and 35 as forward and reverse primer for serotype 15 and SEQ ID NO. 36 as TaqMan probe sequence for serotype 15;
- SEQ ID NO. 37 and 38 as forward and reverse primer for serotype 18B/C and SEQ ID NO. 39 as TaqMan probe sequence for serotype 18B/C;
- SEQ ID NO. 40 and 41 as forward and reverse primer for serotype 19A and SEQ ID NO. 42 as TaqMan probe sequence for serotype 19A;
- SEQ ID NO. 43 and 44 as forward and reverse primer for serotype 19B/F and SEQ ID NO. 45 as TaqMan probe sequence for serotype 19B/F;
- SEQ ID NO. 46 and 47 as forward and reverse primer for serotype 20 and SEQ ID NO. 48 as TaqMan probe sequence for serotype 20;
- SEQ ID NO. 49 and 50 as forward and reverse primer for serotype 22F/A and SEQ ID NO. 51 as TaqMan probe sequence for serotype 22F/A;
- SEQ ID NO. 52 and 53 as forward and reverse primer for serotype 23F and SEQ ID NO. 54 as TaqMan probe sequence for serotype 23F;
- SEQ ID NO. 55 and 56 as forward and reverse primer for serotype 33 A/F and SEQ ID NO. 57 as TaqMan probe sequence for serotype 33 A/F;
- SEQ ID NO. 58 and 59 as forward and reverse primer for serotype 35B and SEQ ID NO. 60 as TaqMan probe sequence for serotype 35B;
- SEQ ID NO. 61 and 62 as forward and reverse primer for serotype 38 and SEQ ID NO. 63 as TaqMan probe sequence for serotype 38;
wherein the primers for the control region are obtained from lytA gene.

6. The kit according to claim 5 wherein primer oligonucleotides are grouped in a plurality of reaction environments.


SEQUENCE LISTING

<110>  Azienda Ospedaliero-Universitaria Meyer
       AZZARI, Chiara
       MORIONDO, Maria
       RESTI, Massimo

<120>  Method for Streptococcus Pneumoniae diagnosis and serotyping

<130>  BE 106086

<160>  64

<170>  PatentIn version 3.5

<210>  1
<211>  22
<212>  DNA
<213>  Artificial Sequence

<220>
<223>  synhetic primer

<400>  1
cgtgcggtaa ttgaagctat ga                                              22


<210>  2
<211>  18
<212>  DNA
<213>  Artificial Sequence

<220>
<223>  synhetic primer

<400>  2
tgtggcccca gcaactct                                                   18


<210>  3
<211>  22
<212>  DNA
<213>  Artificial Sequence

<220>
<223>  synhetic primer

<400>  3
cgtgcttgcc cttgtatagg gt                                              22


<210>  4
<211>  23
<212>  DNA
<213>  Artificial Sequence

<220>
<223>  synhetic primer

<400>  4
ggtcagcaga aagtatgcat tgg                                             23


<210>  5
<211>  22
<212>  DNA
<213>  Artificial Sequence

<220>

<223>  synhetic primer

<400>  5
tcgtttatcc agggtctgat ga                                              22


<210>  6
<211>  25
<212>  DNA
<213>  Artificial Sequence

<220>
<223>  synhetic primer

<400>  6
tattggatgt ggtttatcgt gaaga                                           25


<210>  7
<211>  23
<212>  DNA
<213>  Artificial Sequence

<220>
<223>  synhetic primer

<400>  7
tgggatgaca tttctacgca cta                                             23


<210>  8
<211>  20
<212>  DNA
<213>  Artificial Sequence

<220>
<223>  synhetic primer

<400>  8
ccgtcgctga tgctttatca                                                 20


<210>  9
<211>  24
<212>  DNA
<213>  Artificial Sequence

<220>
<223>  synhetic primer

<400>  9
tcctattgga tggttagttg gtga                                            24


<210>  10
<211>  28
<212>  DNA
<213>  Artificial Sequence

<220>
<223>  synhetic primer

<400>  10
ttacgggagt atcttatgtc tttaatgg                                        28


<210>  11
<211>  26
<212>  DNA

<213>  Artificial Sequence

<220>
<223>  synhetic primer

<400>  11
cagcattcca gtagcctaaa actaga                                          26


<210>  12
<211>  29
<212>  DNA
<213>  Artificial Sequence

<220>
<223>  synhetic primer

<400>  12
ttgtctcagc aactctattt ggctgtggg                                       29


<210>  13
<211>  26
<212>  DNA
<213>  Artificial Sequence

<220>
<223>  synhetic primer

<400>  13
aagtttgcac tagagtatgg gaaggt                                          26


<210>  14
<211>  27
<212>  DNA
<213>  Artificial Sequence

<220>
<223>  synhetic primer

<400>  14
acattatgtc crtgtcttcg atacaag                                         27


<210>  15
<211>  21
<212>  DNA
<213>  Artificial Sequence

<220>
<223>  synhetic primer

<400>  15
tgttctgccc tgagcaactg g                                               21


<210>  16
<211>  19
<212>  DNA
<213>  Artificial Sequence

<220>
<223>  synhetic primer

<400>  16
gatggcatgt ggcaaacca                                                  19

<210>  17
<211>  23
<212>  DNA
<213>  Artificial Sequence

<220>
<223>  synhetic primer

<400>  17
tttgccctcc ttaatcattt cac                                               23


<210>  18
<211>  20
<212>  DNA
<213>  Artificial Sequence

<220>
<223>  synhetic primer

<400>  18
ttggctatcg gcatggtggt                                                   20


<210>  19
<211>  25
<212>  DNA
<213>  Artificial Sequence

<220>
<223>  synhetic primer

<400>  19
ccactcatca gtttcccata tgttt                                             25


<210>  20
<211>  24
<212>  DNA
<213>  Artificial Sequence

<220>
<223>  synhetic primer

<400>  20
tcaataattg aagaagcgaa cgtt                                              24


<210>  21
<211>  24
<212>  DNA
<213>  Artificial Sequence

<220>
<223>  synhetic primer

<400>  21
tgatggcaga tgggttggga cgag                                              24


<210>  22
<211>  21
<212>  DNA
<213>  Artificial Sequence

<220>
<223>  synhetic primer

<400>  22

tggaatgggc aaagggtagt a                                                21

<210>  23
<211>  21
<212>  DNA
<213>  Artificial Sequence

<220>
<223>  synhetic primer

<400>  23
tcggttcccc aagattttct c                                                21

<210>  24
<211>  24
<212>  DNA
<213>  Artificial Sequence

<220>
<223>  synhetic primer

<400>  24
ttaatcatgc taacggctca tcga                                             24

<210>  25
<211>  34
<212>  DNA
<213>  Artificial Sequence

<220>
<223>  synhetic primer

<400>  25
cctctcctat caactattac tcattatact acct                                  34

<210>  26
<211>  30
<212>  DNA
<213>  Artificial Sequence

<220>
<223>  synhetic primer

<400>  26
aataaccata agtccctaga tcattcaaag                                       30

<210>  27
<211>  33
<212>  DNA
<213>  Artificial Sequence

<220>
<223>  synhetic primer

<400>  27
tcattacaac tccctatgtg acacgggtct ttt                                   33

<210>  28
<211>  23
<212>  DNA
<213>  Artificial Sequence

<220>

<223>  synhetic primer

<400>  28
gattattcgc ttgcctcttc atg                                              23


<210>  29
<211>  24
<212>  DNA
<213>  Artificial Sequence

<220>
<223>  synhetic primer

<400>  29
atagccgaaa taagctttcc agaa                                             24


<210>  30
<211>  22
<212>  DNA
<213>  Artificial Sequence

<220>
<223>  synhetic primer

<400>  30
atttgtaagc ggacgtgcga tt                                               22


<210>  31
<211>  26
<212>  DNA
<213>  Artificial Sequence

<220>
<223>  synhetic primer

<400>  31
cgactgaaat gtcactagga gaagat                                           26


<210>  32
<211>  29
<212>  DNA
<213>  Artificial Sequence

<220>
<223>  synhetic primer

<400>  32
aatacagtcc atcaattact gcaatactc                                        29


<210>  33
<211>  31
<212>  DNA
<213>  Artificial Sequence

<220>
<223>  synhetic primer

<400>  33
tgtcattcgt ttgccaatac ttgatggtct c                                     31


<210>  34
<211>  27
<212>  DNA

<213>  Artificial Sequence

<220>
<223>  synhetic primer

<400>  34
ttgaatcagg tagattgatt tctgcta                                          27


<210>  35
<211>  30
<212>  DNA
<213>  Artificial Sequence

<220>
<223>  synhetic primer

<400>  35
ctctaggaat caaatactga gtcctaatga                                       30


<210>  36
<211>  22
<212>  DNA
<213>  Artificial Sequence

<220>
<223>  synhetic primer

<400>  36
ctccggcttt tgtcttctct gt                                               22


<210>  37
<211>  23
<212>  DNA
<213>  Artificial Sequence

<220>
<223>  synhetic primer

<400>  37
cctgttgtta ttcacgcctt acg                                              23


<210>  38
<211>  25
<212>  DNA
<213>  Artificial Sequence

<220>
<223>  synhetic primer

<400>  38
ttgcacttct cgaatagcct tactc                                            25


<210>  39
<211>  22
<212>  DNA
<213>  Artificial Sequence

<220>
<223>  synhetic primer

<400>  39
aaccgttggc ccttgtggtg ga                                               22

<210>  40
<211>  21
<212>  DNA
<213>  Artificial Sequence

<220>
<223>  synhetic primer

<400>  40
ttcgacgacg tatcagcttc a                                                21


<210>  41
<211>  24
<212>  DNA
<213>  Artificial Sequence

<220>
<223>  synhetic primer

<400>  41
tcattgagag ccttaacctc ttca                                             24


<210>  42
<211>  26
<212>  DNA
<213>  Artificial Sequence

<220>
<223>  synhetic primer

<400>  42
acccaaaacg gttgacgcat tatact                                           26


<210>  43
<211>  22
<212>  DNA
<213>  Artificial Sequence

<220>
<223>  synhetic primer

<400>  43
ggtcatgcga gatacgacag aa                                               22


<210>  44
<211>  22
<212>  DNA
<213>  Artificial Sequence

<220>
<223>  synhetic primer

<400>  44
tcctcatcag tcccaaccaa tt                                               22


<210>  45
<211>  28
<212>  DNA
<213>  Artificial Sequence

<220>
<223>  synhetic primer

<400>  45

acctgaagga gtagctgctg gaacgttg                                          28

<210>  46
<211>  28
<212>  DNA
<213>  Artificial Sequence

<220>
<223>  synhetic primer

<400>  46
aaagatactg gctgaggagc tatctatt                                          28

<210>  47
<211>  32
<212>  DNA
<213>  Artificial Sequence

<220>
<223>  synhetic primer

<400>  47
agtcaaaagt actcaaccat tctgatatat tc                                     32

<210>  48
<211>  27
<212>  DNA
<213>  Artificial Sequence

<220>
<223>  synhetic primer

<400>  48
aggataaggt ctactttgtg ggagttc                                           27

<210>  49
<211>  24
<212>  DNA
<213>  Artificial Sequence

<220>
<223>  synhetic primer

<400>  49
tctctgaaat ggttgttgaa ggaa                                              24

<210>  50
<211>  22
<212>  DNA
<213>  Artificial Sequence

<220>
<223>  synhetic primer

<400>  50
tcgcatccga tagttcttgt ga                                                22

<210>  51
<211>  27
<212>  DNA
<213>  Artificial Sequence

<220>

<223>  synhetic primer

<400>  51
caatggctta ctggcaatcc caggaca                                          27


<210>  52
<211>  22
<212>  DNA
<213>  Artificial Sequence

<220>
<223>  synhetic primer

<400>  52
tgctatttgc gatcctgttc at                                               22


<210>  53
<211>  22
<212>  DNA
<213>  Artificial Sequence

<220>
<223>  synhetic primer

<400>  53
agagcctccg ttgtttcgta aa                                               22


<210>  54
<211>  23
<212>  DNA
<213>  Artificial Sequence

<220>
<223>  synhetic primer

<400>  54
tttctccggc atcaaacgtt aag                                              23


<210>  55
<211>  26
<212>  DNA
<213>  Artificial Sequence

<220>
<223>  synhetic primer

<400>  55
cgagagagaa tatgagggaa ttgtta                                           26


<210>  56
<211>  21
<212>  DNA
<213>  Artificial Sequence

<220>
<223>  synhetic primer

<400>  56
tctcaatccc cgcatttact g                                                21


<210>  57
<211>  23
<212>  DNA

<213>  Artificial Sequence

<220>
<223>  synhetic primer

<400>  57
aggaaaactg tggtcacggt tcg                                              23


<210>  58
<211>  20
<212>  DNA
<213>  Artificial Sequence

<220>
<223>  synhetic primer

<400>  58
gcatggaggt ggagcataca                                                  20


<210>  59
<211>  28
<212>  DNA
<213>  Artificial Sequence

<220>
<223>  synhetic primer

<400>  59
tgtaaagact gcacaactcg atataaaa                                         28


<210>  60
<211>  38
<212>  DNA
<213>  Artificial Sequence

<220>
<223>  synhetic primer

<400>  60
caatttaaac aatattagta aagcgcaggt caagcaaa                              38


<210>  61
<211>  26
<212>  DNA
<213>  Artificial Sequence

<220>
<223>  synhetic primer

<400>  61
gtcttacgta gaacctctct ggatga                                           26


<210>  62
<211>  21
<212>  DNA
<213>  Artificial Sequence

<220>
<223>  synhetic primer

<400>  62
tggtcctaca agcgacatgt g                                                21

<210>  63
<211>  29
<212>  DNA
<213>  Artificial Sequence

<220>
<223>  synhetic primer

<400>  63
ttgccacaga tttggaatat tttggtcgg                                          29


<210>  64
<211>  22182
<212>  DNA
<213>  Streptococcus pneumoniae

<400>  64
cgaccgtcgc ttcctagttg tggctaactt gtccaatgaa gagcaagact tgacagtaga        60

aggaaaagtc aaatctgtct tgattgaaaa caccctagct caagaagtct ttgaaaaaca       120

aatcttagtt ccatgggatg ctttctgtgt ggaattacta taaatatttt ttgcagaaaa       180

atttaaaatt gaaatcatat aaaaacaagg gaggactgta taaaagcaga aatcctttgt       240

tttttataac caaggtttat aaactttcat tctcgaaatt caattaactt tacaaattcc       300

cactattaag gagaaagaag atgaacataa agaagcgtgt ccttagtgca ggcctgactt       360

ttgcatctgt tttgctttta gctgcttgcg gccaatcagg ttcagataca aaaacttact       420

catcaacctt tagtggaaat ccaactacat ttaactatct attagactat tacactgata       480

atatagtcaa ttgaaacaag aacaagacaa aagagcctca taaaaggtat tgcaacttgg       540

taataccttt ttgaggtgct ttttgatatg agcccatgtt ttctcaatag gattgtactc       600

aggtgagtag ggaggaagag gtaaaagctt aggctctttg tcaactgtag tgggttgaag       660

tcagctaagc tcgagaaagg acaaatttcg tcctttcttt tttgatgttc agagcgatga       720

aaatccgttt tttgaagttt tcaaagtttc gaaaaccaaa ggcattgcgc ttgataagtt       780

tgatgagatt attggtcgct tccagtttgg cgttagaata gtgtagttga agggcgttga       840

caatcttttc tttatctttg aggaaggttt taaagacagt ctgaaaaata ggatgaacct       900

gcttaagatt gtcctcaata agtccgaaaa atttctctgg ttccttattc tggaaatgaa       960

aaagcaagag ctgatagagt tgatagtggt gtttcaagtc ttccgaataa ctcaaaagct      1020

tgtttaaaat ctctttattg gttcaatgca tacgaaaagt aggacgataa aaatgtttat      1080

cactcagttt acgactatcc tgttgaatga gtttccagta gcgcttgata gccttgtatt      1140

catgggattt tcgatgaaac tgatttatga tttgaacacg cacacgactc atagcacggc      1200

taagatgttg tacaatgtga aagcgatcca acacgatttt agcattcggg agtgaaacag      1260

tctgggagac tgtttcagcc tgagcctaga aatttgaaag cgaaactgtt tagccaagtc      1320

atagtaaggg ctaaacatat ccatagtaat gattttgacg cgacatcgga caactctatc      1380

gtagcgaaga aagtgatttc gaatgatagc ttgtgttcta ccctcaagaa cagtgatgat      1440

attgagattg ttaaaatctt gcgcaatgaa gctcatcttt ccctttgtaa aagcatactc      1500

atcccaagac ataatctcag gaagacaaga aaaatcatgt ttaaagtgaa aatcattgag      1560

cttacgaata acagttgaag ttgagatgga aagctgatgg gcaatatcag tcatagaaat 1620
cttttcaatt aacttttgag caatcttttg gttgatgata cgagggattt ggtgattctt 1680
cttgacgata gaagtttcag cgaccatcat ttttgaacag tgatagcact tgaatcgacg 1740
ctttctaagg agaattctag taggcatacc agtcgtttca agataaggga tcttagacgg 1800
tttttgaaag tcatatttct tcattagact tccacaatca gggcaagatg gagcctcata 1860
atccagctta gcgataattt ctttgtgggt atccatattg atgatatcta gaatcttgat 1920
gtttgggtct ttgatatcga gtagttttgt gataaaatgt aattgtccca tatgaatctt 1980
tctaatgagt tgtttggtcg cttttcatta taggtcatat gggacttttt ttctacaata 2040
aaataggctc cataatatct ataagggatt tacccactac aaatattata gagccaaagt 2100
ttatacccaa actcctcgca taaaagttct aacttaccca ttctatggaa tcttgcatta 2160
tccataataa taaccgatgg tgtggttaat gttggtaaga gaaacttctg aaaccaagct 2220
tcaaaaaagt cgctcgtcat cgtctcttcg taagtcattg gagcgattaa ctcaccattc 2280
atttgttaga cctgcaacca aagaaattct ctgatatctt cttccagata ctttgcctct 2340
tcttaactga ccttttaatg agcgaccata ttctcgataa aaataagtat cgaatcctgt 2400
ttcgtcaatc taaacaggtg ctaggtgctt taaactatta aaattcttaa gaaataaggc 2460
tactttttct gggtcttgtt catagtaggt gtggttcttt tttcgagtgt agcccatagc 2520
tttgagcgca tagtggatgg tagttggatg acagccaaag tcagaagcta tttcagtcaa 2580
ataagcgtct ggattgtcag taagatagtt tttaagtcta tctctatcaa cttttcttgg 2640
ttttgttcct tttacttggt ggtttagctc tcctgttttc tcttttagct ttaaccagcc 2700
ataaatggta ttacgtgaga tttggaaaac gtgtgatgct tctgttatac tacctgttcg 2760
ctcacaataa gagagaactt ttttacgaaa atctattgaa tatgccataa gaagattata 2820
ccacattgtg tactatatta tattgaaact agaatagtac acatctgatt ctaaaacatt 2880
gttagaaatc gatttgactg tcctgattga tttgtcctat tcttatttca ttttactata 2940
tttttggttc gcgggaagtc tactaagata cttaaagatg cagatagtga aaataaaggt 3000
gtagacatta ccgtaaaaaa gtgatataat tgtatgatgt tcaaggtata ggtgttaatc 3060
atgagtagac gttttaaaaa atcacgttca cagaaagtga agcgaagtgt taatatcgtt 3120
ttgctgacta tttatttatt gttaatttgt tttttattgt tcttaatctt taagtacaat 3180
atccttgctt ttagatatct taacctagtg gtaactgcgt tagtcctact agttgccttg 3240
gtagggctac tcttgattat ctataaaaaa gctgaaaagt ttactatttt tctgttgctg 3300
ttctctatcc ttgtcagctc agtgtcgctc tttgcagtac agcagtttgt tggactgacc 3360
aatcgtttaa atgcgacttc taattactca gaatattcaa tcagtgtcgc tgttttagca 3420
gatagtaaga tcgaaaatgt tacgcaactg acgagtgtga cagcaccgac tgggactgat 3480
aatgaaaata ttcaaaaact actagctgat atcaagtcaa gtcagaatac cgatttgacg 3540
gtcaaccaga gttcgtctta cttggcagct tacaagagtt tgattgcagg ggagactaag 3600

gccattgtcc taaatagtgt ctttgaaaat atcatcgagt cagagtatcc agactacgca 3660
tcgaagataa aaaagattta taccaaggga ttcactaaaa aagtagaggc tcctaagacg 3720
tctaagaatc agtctttcaa tatctatgtt agtggaattg acacctatgg tcctattagt 3780
tcggtgtcgc gatcagatgt caatatccta atgactgtca atcgagatac caagaaaatc 3840
ctcttgacca caacgccacg tgatgcctat gtaccaatcg cagatagtgg aaataatcaa 3900
aaagataaat taacccatgc gggcatttat ggagttgatt cgtccattca caccttagaa 3960
aatctctatg gagtggatat caattactat gtgcgattga acttcacttc tttcttgaaa 4020
atgattgact tattgggagg ggtagatgtt cataatgatc aagagttttc agctctacat 4080
gggaagttcc atttcccagt agggaatgtc catctagact ctgagcaagc tttaggtttt 4140
gtacgtgaac gctactcact aaccgatgga gaccgtgacc gtggtcgcaa ccaacaaaag 4200
gtcattgtag caattattca gaagttaact tctacagagg ttttgaaaaa ctatagtagt 4260
attcttcaag gattgcagga ttctcttcaa acaaatatac cgattgagac tatgatagat 4320
ttagtgaata ctcagttgga aagtggaggg aattataaag taaattctca agatttaaaa 4380
gggacaggtc ggatggatct tccttcttat gcaatgccag acagtaacct ctatgtgatg 4440
gaaatagatg atagtagttt aactgtagtt aaagcagcta tacaggatgt gatggagggt 4500
agataaaatg atagacatcc attcgcacat cgtctttgat gtagatgatg gtcccaagtc 4560
aagagaggaa agcaaggctc tcttggcaga atcctacagg cagggggtga gaaccattgt 4620
ctctacctct caccgtcgca agggcatgtt tgaaactccg gaagagaaga tagcagaaaa 4680
ctttcttcag gttcgggaaa ttgcaaaaga agtagcagat gatttagtca ttgcttatgg 4740
cgcagagata tactatactc tggatgctct agaaaagcta gaaaaaaaag aaattcctac 4800
ccttaatgat agtcgttatg ccttgattga gtttagcatg catacttcct atcgtcagat 4860
tcatacggga ttgagcaata ttttgatgtt gggaatcacg ccagtaattg ctcatattga 4920
acgttatgat gctttagaga ataacgaaaa acgtgttcgt gaactgattg atatggggtg 4980
ctatactcag ataaatagtt atcatgtttc aaaacctaag ttctttggtg aaaaatataa 5040
attcatgaaa aagagagctc ggtatttttt ggaacgtgat ttagttcatg tagttgcaag 5100
tgacatgcac aatttagaca gtagacctcc atatatgcaa caggcatatg atatcattgc 5160
taagaaatat ggagcgaaaa aagcgaaaga actttttgta gataatccca gaaaaattat 5220
aatggatcaa ttaatttagg agaaaatatg aaggaacaaa acactttgga aatcgatgta 5280
ttgcaactat tcagagcttt atggaaaaga aagttggtca ttttattagt ggcaattata 5340
acttcttcag ttgcttttgc ctacagtact tttgttatca aacctgagtt tactagtacg 5400
actcggattt atgtagttaa ccgtaatcag ggagagaagc ctggtttaac caatcaagac 5460
ttgcaggcag gagcatactt ggttaaagac tatcgtgaaa ttgtcctatc gcaggatgtt 5520
ttggaggaag ttatttctga tttgaaacta gatttggtgc caaaaggttt ggctaataaa 5580
attaaagtaa cagtaccagt tgatacccgt attgtctctg tttcagttag tgatcgagtt 5640
cctgaagagg caagccgtat cgctaactct ttaagagaag tagctgctca aaaaattatc 5700

agtattactc gtgtttctga tgtgacaaca ctggaggagg caagaccggc gatatcaccg 5760
tcttcgccaa atattaaacg caatacacta attggttttt tggcaggggt gattggaact 5820
agtgttatag ttcttcttct tgaacttttg gacactcgtg tgaaacgtcc ggaagatatc 5880
gaaaatgcac tgcagatgac acttttggga gttgtaccaa acttgggtaa gttgaaatag 5940
gagagagaga tgccgacatt agaaatagca caaaaaaaac tggagttcat taagaaggca 6000
gaagaatatt acaatacctt gtgtacaaat atacagttga gcggagataa actaaaagta 6060
atttccgtta cttctgttaa tccgggggaa ggaaaaacaa ctacttccgt aaatatagca 6120
atgtcatttg cgcgtgcagg ctataaaact cttttgttcg atggcgatat tcgaaattca 6180
gttatgtcag gattttttaa atctcgtgaa aaaattacag ggctaacaga atttttatct 6240
gggacagctg atttatctca cggtttatgt gatacaaata ttgaaaattt atttgtagtt 6300
caatcgggat ctgtatcacc aaaccctaca gccttgttac aaagtaaaaa ttttaatgat 6360
atgattgaaa cattgcgtaa atattttgat tatatcattg ttgatacacc acctattgga 6420
attgttattg atgcggcaat tatcactcaa aagtgtgatg cgtccatctt gataacagca 6480
acaggtgagg cgaataaacg tgatgtccaa aaagcgaaac aacaattaga acaaacaggg 6540
gaactgttcc tgggagttgt tttaaataaa ttggatatct cggttgataa gtatggagtt 6600
tacggtcgta aatagtttgt aagttctata tgtgtataga attttaaagt gtcacaattt 6660
ggaaacaatt acttatagaa aattactatt tttaaaatga tagttgtaaa ggataatgga 6720
gtagcggtta attatatttt aggaagatta ttttgatgaa tttacaaaag aaaaaaatta 6780
tgttaataac taatcatgac gatgatatct attgttttcg aaaagaagtt atagagtcac 6840
tagttgagta tgggtatgaa gtgctagtgt catgtcctta tggagataag atagagttga 6900
tgaagtctat tcaatttgag tatgaagata ttactattga tagaagagga ataaaccctt 6960
taaaagatat tcgtctattt cttagttata taagattatt gagaaaatat agaccggatg 7020
tagtcttgac atataccgtg aaaccaaatg tatatgctag cctagctgca acaattttag 7080
gaataaaata tataaataac gtaacaggtt taggtagtgt cttaacaatg ggttcttgtg 7140
taagaagcct ggtgttatta ctgtttagaa tttcttttag gaaatcaaat tgtgtttttt 7200
ttcaaaatga atataatatg aaaatgttaa aaactcttgg cttgattttg ggtgactcta 7260
agctgatacc tgggtcagga gtgaacattg aaaagtatcc tgtgcaacag tatccaaatg 7320
gtggaaatgg aattcaagga gaaacaatcg tttttaattt tattggtagg atattaaaag 7380
aaaaaggtat agatacttat ctggctgctg cccaaattat taagagtcga tatcccaaaa 7440
cagagtttaa tattattggc tttatagaac cgacagagag taattatgaa cttaaaattt 7500
gtgacttaga aaaaaaagga atcgtttatt atttgggaca acaaaaagat gtgatacctc 7560
atattacccg ttcccatgca attatccatc ccagtgtgta tggtgaagga atgagcaatg 7620
tattactaga aaacgctagt tcaggacgtg ttttaattac gacagataat ccaggttgca 7680
aagaaattgt taaagataga gagacaggct atatatttca agggggaaat gttgaggaac 7740

tagtctctat attggaagtt tttttaggtc tagaaaatga aaaacgaaaa gagatgggac 7800
ttcaagggag aatgtatata gagcaatatt tttctcgtca aattattata gatgcttatt 7860
tagataaaat aaaaggagct ttagatgaat aggattataa aaaatattgt tggtttactt 7920
cgtgcaaata tttgcttgat attattaaaa gtgtgtcgaa acaaggatct tacttttcat 7980
tattttttaa aatttggaaa gaatgtaact gtcgaacagg aatctaatgc taaattaatc 8040
ttaggaaaaa agattagagt aaacgccggg ggagtattga aagttagaaa aggagcaaaa 8100
ctcaagattt ctgatgatgt atttttgagt aataattgta tgatagcttg tcgtaaatac 8160
atagatatta aatctggagt aaaatgtggt cctggagtac ttatatatga tcatgactat 8220
gatgttagtg ttccaggtgg attgaaagca aaaaaattta agacggcccc agttatgatt 8280
ggagaaaatg tttggattgg agctaacagc attgtcttga agggagtgag tattggtgag 8340
aatagtgtgg ttgcagcagg aagtgttgta acaaaggata ttccagctga tactatattt 8400
attcagaaac gtttatcaag ggagatgaaa ttatgaaagt atttatagcg actgaattta 8460
gatgtacaat ctataaaaat gaaatatatt taaaaccgaa agcttatttt atttatgagc 8520
gctatgctaa agcatttgga gagattgtac tatgtagtcg ttatgagaag gtggaatcat 8580
accctgaagg atatcataaa gcgaacttta ttaataaatt tatccctata gaaggtctac 8640
atcaggttct atttggtcaa aataaaaata agattattga aggaatgatt gatagcgact 8700
taatagttgt tcgtattccg tctataattg gatcaaaaac tgcagactac gcattgaaga 8760
taggtaagcc gtatctgaca gaaataatgg gggatgcttg ggattcttac tggtatcata 8820
gtttaaaggg aaaattatta gctccatata tatacgccaa aactaaatca attgtaaaaa 8880
acgctaatta ttgcatatac gtgacagaaa aatatttaca agatagatat cctaatatta 8940
aatctaatat cgttgcttca aatgttaata ttacctctgt agagaataga tctttgaaga 9000
gccgtcttta taagttgaaa aaatttaatc ctcaaaaaat ttcaataatg acaacagcat 9060
ctgtgaatgt acgagccaag ggccatagat ttgtattgga agcaatgaag agattagaaa 9120
tacaaggtat tttgttggat tattatttag caggtgatgg tgatcaaagt ttcttaaaaa 9180
agaaagcaga ggaattggga gtagcgaata gaatccattt tttaggggaa ttgactacat 9240
cacaaattta tgagtatttg gataaagttg atttatatat acaaccaagt ttacaagaag 9300
ggttacctcg tgcggtaatt gaagctatga gccgtgcttg cccttgtata gggtctaata 9360
aagcaggaat tccagagttg ctggggccac attgtctatt tactccatcc tcttcacaag 9420
cgattgcggg atctattata gattttatta gtatggataa ggaaaagatg gtgataaaac 9480
agtttgaatt ctcaaaaaaa tttctttcga gtgtattaaa taaacgcaga agtgactatt 9540
ttgctcaaat tagaagagaa ttaaataatt ctgatggttt agattaaaat gcgatggaaa 9600
atccgtttcg atatattaaa tatgctataa aaactgacta catagccaat caaatttttg 9660
aatacgcatt acgtttaaat tattagttaa aggaaaaaag taaatgaagt ttagtctaca 9720
atcaaatgat ataaaaaata aaataatact tctagtaggt gtagtttttt cagggacttt 9780
aattttatgc agtgaaaata atgatgttac aattccatta ttttcaatga tttttttaag 9840

ttttatggca atgataattg tgaaatttaa tatttttcat ccctatgtgt ggtatagtag 9900
cttctttact atttattcta tttcttaccc gctactctat agaatggagt atataaacta 9960
tggttataca aaagatttga ttttcatgca atggctggct ttggcaactt tgttgttaac 10020
tctaccttct aaagccattg tgttaagcga aaggaaaata aacctaaata acaataacta 10080
tttattattt ctcaataata tgaatacgtt tatcacaaca gtagcaatta tatatctatt 10140
aggaagtggt tttagaaata aaggggaaat atattcaggt gccaatattg tgataatgtt 10200
agtattttct ttggtttatt ttatgattct cattcataca tatcagctat ttatacagtt 10260
gagtactaaa aaatggagac tactaaattg taatactaac acagcgatag ttatctgtct 10320
cttctctatc gttacagggg aaagagatta tatgtttact ttattcatcc ttgatgtgat 10380
tatattattt tactttcaca aaataaaacg tttttatctt gtaatgttag ttccgcttgc 10440
ggcactactg attccgttat ctgcagtttt taaatatact ttattaagtg ggcaagtttc 10500
atctataaat acaaataata tttggtttga tttgctagat ggtgagtttg tatcagctag 10560
tcgtaattta cagattctga tattacataa tatgggcaat tatttcgaag gtcgttcttt 10620
ttttaatgac atagtaagaa ttttttataa tacggggtat tctaatcaaa cttggtttat 10680
ggacaccttt tttccaaacg tacattcaac caaatatggt tttactcttg tgggagaggg 10740
atatgtgaat ggaggatact ttggaattgt aatgattttt atgctaacag gctttttgat 10800
gagatttctt tatataaacg ctcaaagaaa tatatacggg atgctgatat atctgtatat 10860
gatacctatt tttatttatt ctactcgtgc tgattttgct aacatattat ctccattact 10920
taaatatgct atattgggaa cattggttat tgtttttatc aatagaatgg tgttgatatc 10980
accattggat gaaatgggtg gtgagtagtt tatttaaaaa ggtggggtat actttttgat 11040
actatagtgg tttgaagttg gagtagtaca atgcagcttc taaagaattt ttataaatta 11100
ctttaaattc cctaatcgtt ttgttcatat tctatttcaa tatactataa ttttaaattt 11160
actgttataa aatttaacat acggagaaaa taagtgagac tatctaagaa atattggttt 11220
gctttaacga gtaaattttt gttaatctta ttaacacttt tatcctcaat tataattaat 11280
aggggattag gtgttgaaaa aaaaggagat tatgcctatg taatgaactt agtagaaatt 11340
ttttatattt taggtggagt tggtttaggt caggcttact cgacctttaa aaggaatcat 11400
tctaatgagt atcagaataa atttgttttt ttaactttta ttcaaggatt attgtttgga 11460
ataataggtt taataatagt tttagctgat gttccgatag attatggtgg ggtaataata 11520
gttttgtcca gtctatctat tatgagaaca aatcttacta tgattgcagt aatagaaaat 11580
agtataaaga ggaataagat tgtattagga gtaagcgtag tatattccgt tatgctagta 11640
ttgttgttac tatttcattt agtttctata gagatggtat ttttggtata tgctattaat 11700
gaatttttac gtatcatttt atttttatac tattacaata tgttcccagt ttttgaacat 11760
attagtatta aagaattttt cactatatat aagtttggat ttctttctat gattcttact 11820
ctattaatta catttaatta tagtattgat atcataatgt tgaagcaaat gtctagtagt 11880

tataatgtag gtatatacag tgtcgcagtt acgttttcta atatgttttt gatggttcca 11940
gactcattta aagagatttt atttggtgat tcggctaaaa gaacttttga gaaaaataaa 12000
gcaataaatg caataaaagt atcatttcta tttatgattt tattaattat tggatttctt 12060
tcttttggag aatttgcgat tacgctcctt tatggttcca gttatataaa ggcatttcct 12120
ataacattag tcattttttt aggaagctta tcgatgattt tctttaaaat tttacaacca 12180
atttatatat ctcagggtaa acagagtgta gctataaaaa ttttatctgt gtcggctttg 12240
ataaatataa gtttaaactg gtacttaatt cctaactacc aaattacagg agctgcgata 12300
gcatcagcaa tctcttatac tgtatgtggt ttgtttttta ttatagatta tcttagaaat 12360
tgattataat agttgttaaa gaatgtgtat tagtggatga aaatataaaa ataacaagag 12420
gtattatgat atgagaggaa aaacaattct aattacagga gtagctggtt ttattggatc 12480
aaatctggtt aggaaattat tggaattaga aggaacgatg acaatcattg gtttggacaa 12540
tttgaatgat tactatgata ttgcacttaa agattatcga ttgcttcaac ttaataagct 12600
aattcaggat tatccagaaa aaaactggat atttatcaag ggtgatattg cagataaaat 12660
gcttattaat aagattttta agagttatca tcctcagatt gttgttaatc tagctgccca 12720
ggcaggagta cgctactcca ttacaaatcc agatgtatat atagaaagta atatcgttgg 12780
ctttcataat gtgttggaag cttgtcgtca ttcgtatgag atatatgatg gtggtgtgga 12840
acacttagtt tatgcatcgt cctcatcggt atatggtgga aataaaaaaa tcccgtttgc 12900
aacagaagat aaggtagaca atcctatttc actatatgct gcgactaaaa aaacaaatga 12960
acttcaagcg tatgcttatt caaagattta caatattcct tcgacaggct tacgcttttt 13020
tacagtttat ggtcctgctg ggcgcccaga tatggcctat tttggattta cagataaatt 13080
gcgaagtggt tccgatattg agatttataa tcatggtaat tgtaaacgag attttactta 13140
tatagatgat atcgttgagg ggatagtaag ggtcatgcaa tcagctccga agaaactagt 13200
gggatctgat aatttaccgt tagccccata taaagtgtac aatattggga atagtaaacc 13260
cgaaaatttg ttagattttg tggatgttct tcaacaggag ctaatcaagg caggagtttt 13320
gccggaaaat tatgattttg atagtcataa aaaattagtt cctatgcaac caggcgatgt 13380
tcctgttact tatgcagata caagtgattt agagagagat tttggtttta aaccaaaaac 13440
ttctttacga gaaggtttga gaaagtttgc agagtggtat aaagactact ataatgtata 13500
acgagagtgt tttgagaaag gaaaaaatta tgaaaatagc agtagcaggt acaggatatg 13560
ttggattatc tattgcaatc ttactggctc aacatcatca agttgttgcc gtagatatca 13620
ttcctgagaa agttgagtta atcaataaaa agaagtcccc tatccaagac gattatatag 13680
aaaaatatct cgcggaaaaa gaattgaatc tcgtagctac attgaatgca gaggaagcat 13740
atgagaatgc ggattttata gttattgcgg ctccaactaa ttatgatccg aaaaaaaatt 13800
ttttcgatac atcttcagtc gaggctgtca ttgaagcagt tatgaaatat aatcctgatg 13860
caattataat aattaaaagt actgttcctg ttggatatac aaagtcagta cggaggaaat 13920
ttcttacaga caatattatg tttagtcctg agtttttacg ggagtcaaaa gcgttatatg 13980

ataacctata tccgagtcga attattatag gtacagataa agatgataaa gatttggtaa 14040

agtctgctga aatttttgta aaaatgcttc aggaaggtgc agtgaaagaa aatattgaaa 14100

cattgtatat ggattttaca gaagctgaag cagtaaaact ttttgctaat acctatcttg 14160

ccctccgggt ttcctatttt aacgagttag atacttattc tgagatgaaa ggactagata 14220

ctcaagcgat tattgatgga gtttgtttag atccgagaat aggttctcac tacaataatc 14280

cttcttttgg ttatgggggt tactgtcttc ctaaagatac caagcaactt cttgcaaatt 14340

attctgacgt tccacaagaa atgataacag ctatagtaca aagtaatcgg acaagaaaag 14400

attatattgc agatcgtatt ttagatcttg ctggatctta tgtaggcaat gatttctata 14460

aaccagataa agaaaaagag attgttgtag gaatttatcg tttaacaatg aaaagtaatt 14520

cagataattt tagacaatct tctattcaag gtattatgaa gcgtttgaaa gcaaaaggtg 14580

ttactattat tatttacgaa ccaactcttg aaagtggtac tactttctat ggtagtcaag 14640

tagttaatga ttttgaaata tttaagaaat ctagtcaagt gattgttgca aatcgttttg 14700

atgagacttt agaagatgtg aggggtaaag tttatactag ggatattttt aagagagatt 14760

aaatcaattt gtatatttct agtccctaat aatctatatc atttgaatga tagaatagtt 14820

aaaaactagc tggaggatag cgacaactca tctaaaacaa taaagatatt tctatattct 14880

cgctcgcact tagtgaagta aatatacttc ttttgtcagt agagattgac ttaataggat 14940

atagttgcat atattccttc gaagtgaggt gctgataatg gcagtaattt ttaaaaatgt 15000

gcttattgaa gagaagactg aagaagaaaa acaacatatt ctatctattc taaatggtga 15060

gtatgactta tctgatgatg taagttgtag attacgacga ctgaatgagg aaagtaaacg 15120

cttggaagtg tttgttaagg agacgaactc acttgtctag ttcgtattct gttaatgaat 15180

tgaaatcaat aagtatctca ttgtcatttt ttaactatga ggaagttaga gatttctaat 15240

agctctccag ttactatgta atttagcatt gaaaaactaa gaaatggtga aattatgcct 15300

tttacacagt tatttaaaac atccttagga attgattttt aattgtatgc aaacaaagta 15360

gaattcgctt atttcatttc atgctaaatg taatatgaag taagtgaatt tttaggctct 15420

ttgtcaactg tagtgggttg aagtcagcta agctcgagaa aggacaaatt tcgtcctttc 15480

ttttttgatg ttcagagcga tgaaaatccg ttttttgaag ttttcaaagt ttcgaaaacc 15540

aaaggcattg cgcttgataa gtttgatgag attattggtc gcttccagtt tggcgttaga 15600

atagtgtagt tgaagggcgt tgacaatctt ttctttatct ttgaggaagg ttttaaagac 15660

agtctgaaaa ataggatgaa cctgcttaag attgtcctca ataagtccga aaaatttctc 15720

tggttcctta ttctggaaat gaaaaagcaa gagctgatag agttgatagt ggtgtttcaa 15780

gtcttccgaa taactcaaaa gcttgtttaa aatctcttta ttggttcaat gcatacgaaa 15840

agtaggacga taaaaatgtt tatcactcag tttacgacta tcctgttgaa tgagtttcca 15900

gtagcgcttg atagccttgt attcatggga ttttcgatga aactgattta tgatttgaac 15960

acgcacacga ctcatagcac ggctaagatg ttgtacaatg tgaaagcgat ccaacacgat 16020

tttagcattc gggagtgaaa cagtctggga gactgtttca gcctgagcct agaaatttga 16080
aagcgaagct gtttagccaa gtcatagtaa gggctaaaca tatccatagt aatgattttg 16140
acgcgacatc ggacaactct atcgtagcga agaaagtgat ttcgaatgat agcttgtgtt 16200
ctaccctcaa gaacagtgat gatattgaga ttgttaaaat cttgcgcaat gaagctcatc 16260
tttccctttg taaaagcata ctcatcccaa gacataatct caggaagaca agaaaaatca 16320
tgtttaaagt gaaaatcatt gagcttacga atagcagttg aagttgagat ggaaagctgc 16380
tgggcaatat cagtcataga aatcttttca attaactttt gagcaatctt ttggttgatg 16440
atacgaggga tttggtgatt cttcttgacg atagaagttt cagcgaccat catttttgaa 16500
cagtgatagc acttgaatcg acgctttcta aggagaattc tagtaggcat accagtcgtt 16560
tcaagataag gaattttaga aggtttttga aagtcatatt tcttcaattg acttccgcac 16620
tcagggcaag atggagcctc ataatccagc ttagcgataa tttctttgtg ggtatccata 16680
ttgatgatat ctagaatctt gatgtttggg tctttgatat cgagtagttt tgtgataaaa 16740
tgtaattgtc ccatatgaat ctttctaatg agttgtttgg tcgcttttca ttatagatct 16800
tatgggactt tttttctaca ataaaatagg ctccataata tctatagtgg atttacccac 16860
tacaaatatt atagagccat ataaaacatg ggatgattgt tttcttttta gagcagtaca 16920
caattaatag taattgactc taaaataaca aagaaaggta ccctactatg aaaggtatta 16980
ttctagcagg tggttcgggg acacgtttat atcctttgac tcgcgctgcg tcaaaacaac 17040
ttatgccggt ttatgataaa ccgatgattt actacccact ttcaacattg atgttggctg 17100
ggattaggga tattttgatt atttccactc cacaggattt acatcgattt caagagcttc 17160
ttcaagacgg atctgagttt gggatcaaac tttcttatgc agagcaacca agtacagatg 17220
gtttggcaat tggggaagag tttatttctg acgatagcgt tgccttgatt ttaggcgaca 17280
atatctacca tggtcctggt ctgagcacaa tgcttcaaaa agcagccaag aaagagaaag 17340
gtgcgactgt ttttggctac caagtgaagg atccagagcg ttttggtgtg gttgagtttg 17400
atacagacat gaatgctatt tccatagaag aaaaaccgga gtatcctcgc tccaactatg 17460
cagtgacagg actgtatttc tatgataacg atgttgtcga aattgccaag agtattaaac 17520
caagccctcg tggtgaactg gaaattacag atgtaaacaa ggcttaccta gatcgtggtg 17580
atttgtctgt tgagcttatg ggacgtggct ttgcttggct gaatactgga actcatgaaa 17640
gtttactaga ggcttcacag tacatcgaaa cagtgcaacg gatgcaaaat gttcaggtag 17700
caaacttaga agaaattgct taccgtatgg gctatatcag tcgagaagat gtattggcct 17760
tagcccaacc acttaagaaa aatgaatacg gacagtatct gctccgtttg attggagaag 17820
catagatgac agataatttt ttcggtaaga cgcttgcggc acgcaaggtt gaagctattc 17880
caggcatgtt ggagtttgat atccccgttc atggagataa tcgtggctgg tttaaagaaa 17940
atttccaaaa ggaaaaagtg cttccacttg gatttccaga gtctttcttt gcagaaggaa 18000
aattgcaaaa caatgtatcc ttctcacgta aaaatgtcct tcgaggcctc cacgcagagc 18060
cttgggataa gtacatctct gtagcagatg gagggaaagt tctgggttct tgggttgatc 18120

tacgcgaggg tgaaaccttt gggaatacct atcagacagt aattgatgca agcaagggaa 18180
tctttgttcc tcgaggcgta gctaatggct tccaagtttt atcagataca gtgtcatata 18240
gctatctggt caatgattac tgggctcttg aactcaaacc caagtatgcc tttgtgaact 18300
acgctgatcc aagccttggt attgaatggg aaaatctgga agaagcagac gtatctgaag 18360
cagacaaaca tcatccccta cttaaggacg tgaagccttt gaaaaaagaa gatttggaat 18420
aaggaaagaa tatgactgaa tacaaaaata ttatcgtgac aggtggagct ggctttatcg 18480
gttctaactt tgtccattat gtttacgaga actttccaga tgttcacgtg acagtcctag 18540
ataagttgac ttatgctgga aaccgcgcga atattgagga aattttaggc aatcgtgttg 18600
agttagttgt tggtgacatt gctgatgcgg agttggtaga caagttggtt gctcaagcag 18660
atgctatcgt tcattatgca gcggaaagcc acaatgataa ttcgctcaat gatccatcgc 18720
catttattca tactaacttc attggaatct atactctttt agaagctgct cgtaagtatg 18780
atattcgctt ccaccatgta tcgacagatg aagtttatgg ggatctccct ttacgagaag 18840
atttgccagg tcatggtgaa ggaccaggtg agaaatttac tgctgaaaca aaatacaacc 18900
caagctctcc gtactcatca accaaggccg catcagactt gattgtcaaa gcctgggtgc 18960
gttcttttgg agtcaaggca acgatttcca actgttcaaa taactacggt ccttatcaac 19020
atatcgaaaa attcatccca cgtcagatta ctaacatcct aagtggaatt aagccaaaac 19080
tttacggtga aggtaagaac gttcgtgact ggattcatac caatgaccat tcttcaggag 19140
tttggacaat cttgacaaaa gggcaaatcg gtgaaaccta cttgattggg gctgatggtg 19200
agaagaacaa taaggaagtt ttggaactta tccttaagga aatgggacaa gctacggatg 19260
cctatgatca tgtgactgac cgtgcaggac atgaccttcg ctatgcgatt gatgccagca 19320
agctccgtga tgagttgggg tggaaacctg aatttaccaa ctttgaagct gggctcaagg 19380
caacaatcaa gtggtataca gataaccaag aatggtggaa agcagaaaaa gaagctgttg 19440
aagccaatta tgctaagact caggagatta ttacagtata aaaagcagga aatcgctgct 19500
ttttattgct atattgggaa gagttacata ttagaaaggt ctagagatga ttttaattac 19560
aggggcaaat ggccaattag gaacggaact tcgctattta ttggatgaac gtaatgaaga 19620
atacgtggca gtagatgtgg ctgagatgga cattaccaat gaagaaatgg ttgagaaagt 19680
ttttgaagag gtgaaaccga ctttagtcta ccattgtgca gcctacaccg ctgttgatgc 19740
agcagaggat gaaggaaaag agttggactt cgccatcaat gtgatgggga caaaaaatgt 19800
cgcaaaagca tctgaaaagc atggtgcaac tctagtttat atttctacgg actaggtctt 19860
tgacggtaag aaaccagttg gacaagagtg ggaagttgat gaccgaccag atccacagac 19920
agaatatgga cgcactaagc gtatggggga agagttagtt gagaagcatg tgtctaattt 19980
ctatattatc cgtactgcct gggtatttgg aaattatggc aaaaacttcg tttttaccat 20040
gcaaaatctt gcgaaaactc ataagacttt aacagttgta aatgaccagt acggtcgtcc 20100
gacttggact cgtaccttgg ctgaattcat gatctaccta gctgaaaatc gtaaggaatt 20160

tggttattat catttgtcaa atgatgcgac agaagacaca acatggtatg attttgcagt 20220
tgaaattttg aaagatacag atgtcgaagt caagccagta gattccagtc aatttccagc 20280
caaagctcaa cgtccgctaa actcaacgat gagcctggcc aaagccaaag ctactggatt 20340
tgttattcca acttggcaag atgcattgca agaattttac aaacaagaag tgagataagt 20400
agtagaatga ttttctagtc taataaaaga ggcagataat gaactccaaa ggagcttaag 20460
atgtacaatt atcttgttgt tggtgctggt ctctttggtg catagctttg gctcagtttc 20520
tattatcgct cacaccatcc atcagaagtt taatctgaag gtacccaatt atcgccaaga 20580
agaagattgg gctaggatgg gtttaccaat cacacgtaag gaaatctcta attggcatat 20640
caaggcaagt caatactatt tagagtccct ttataagctt ttacgagaaa agttgttaga 20700
acaacctctt cttcatgcgg atgaaacctc ttatcgggtt ctagagagtg atagccatct 20760
gacctactat tggacttttt tgtctgggaa agctgagaat caagcaatca cgctgtacca 20820
ccatgatcag cgtcggagtg gtttagtagt acaagaattc ctaggagatt attctggcta 20880
tgtgcattgt gatatgttgc ggcagtaact taggacttta gtcctctagt tctgcctatg 20940
cgatagcagt ccaaggttta ggagcaaggc gacgctaagc ttggtaaact gcgaaccgct 21000
agaagcttat cgtcaactgg aagaagctga acttgttgga tgttgggcac atgtgagaag 21060
gaagtttttt gaagcgcccc ccaagcaaac ggataaatca tccttaggag ctaaaggttt 21120
agcttattgt gatcagttat tttccctgga aagagactgg gaggctttgc cagctgatga 21180
acgactacag aaacgtcaag aacatctcca gcccctaatg gaagacttct ttgcttggtg 21240
ccggcgtcag tcagttttag caggttcaaa actaggaagg gcaattgaat acagcctcaa 21300
gtatgaagaa acctttaaga ctattttgaa agacggacat ctggtccttt ccaataatct 21360
agctgaatgc gccattaaat cattggttat gggacggagt aaaagagtcc agtggactct 21420
tttagcctaa gctcagttta aaaaagcgag ggtggttatt ttctcaaagt tttgaaggag 21480
ctaaagcaag agctattata attaaatagc gagaaatatc tatcctatct tctagaatgt 21540
cttccaaacg aggaaactct cgtaaacaaa gagattttag aggcctattt accgtggact 21600
aaagttgtac aagaaaagtg caaataagaa atctccagat taggaactat ccgtgagttc 21660
tctagtctgg agatttttca atagacttcg ttattgggcg gttacgatat tcatattttt 21720
tgcaaagatg ttatttgaaa aataattttc aaaaattctg aaaattctgt tgacaacttt 21780
ctgaaaagag tctataatgg agagaaagtt ttaaaggaga aaaggatgaa aagttcaaaa 21840
ctacttgccc ttgcgggcgt gacattattg gcggcgacta ctttagctgc atggtctgga 21900
tcaggttcaa gcactaaagg tgagaagaca ttctcataca tttatgagac agaccctgat 21960
aacctcaact atttgacaac tgctaaggct gcgacagcaa atattaccag taacgtggtt 22020
gatggtttgc tagaaaatga tcgctacggg aactttgtgc cgtctatggc tgaggattgg 22080
tctgtatcca aggatggatt gacttacact tatactatcc gtaaggatgc aaaatggtat 22140
acttctgaag gtgaagaata cgcggcagtc aaagctcaag ac 22182